(19) Europäisches Patentamt
European Patent Office
Office européen des brevets

(11) Publication number: **0 387 067 B1**

(12) **EUROPEAN PATENT SPECIFICATION**

(45) Date of publication of patent specification: **31.08.94**

(51) Int. Cl.5: **C12P 21/02**, C12N 15/52, C12N 15/54, C12N 1/21, C12P 13/04, //(C12N1/21, C12R1:19)

(21) Application number: **90302496.6**

(22) Date of filing: **08.03.90**

# (54) Novel recombinant DNA and method for production of physiologically active substance.

(30) Priority: **08.03.89 JP 53718/89**
**23.02.90 JP 41296/89**

(43) Date of publication of application:
**12.09.90 Bulletin 90/37**

(45) Publication of the grant of the patent:
**31.08.94 Bulletin 94/35**

(84) Designated Contracting States:
**DE FR IT**

(56) References cited:
**EP-A- 0 107 400**
**EP-A- 0 202 094**

**JOURNAL OF BACTERIOLOGY, vol. 171, no. 1, January 1989, pages 577-580, American Society for Microbiology; A. MATSUYAMA et al.: "Cloning, expression, and nucleotide sequence of the Escherichia coli K-12 ackA gene"**

(73) Proprietor: **KIKKOMAN CORPORATION**
**339 Noda**
**Noda-shi, Chiba-ken (JP)**

Proprietor: **Kimura, Akira**
**81, Kami-wakamiya-cho, Rokujo-agaru,**
**Wakamiya-dori, Shimogyo-ku**
**Kyoto-shi Kyoto-fu (JP)**

(72) Inventor: **Matsuyama, Asahi**
**c/o Kikkoman Corporation,**
**339 Noda**
**Noda-shi, Chiba-ken (JP)**
Inventor: **Otake, Hideko**
**c/o Kikkoman Corporation,**
**339 Noda**
**Noda-shi, Chiba-ken (JP)**
Inventor: **Kitao, Satoshi**
**c/o Kikkoman Corporation,**
**339 Noda**
**Noda-shi, Chiba-ken (JP)**

**EP 0 387 067 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**GENE, vol. 39, 1985, pages 263-267, Elsevier Science Publishers; P.A. BENFIELD et al.: "Expression of a rat brain creatine kinase-beta-galactosidase fusion protein in Escherichia coli and derivation of the complete amino acid sequence of rat brain creatine kinase"**

Inventor: **Nakano, Eiichi**
**c/o Kikkoman Corporation,**
**339 Noda**
**Noda-shi, Chiba-ken (JP)**
Inventor: **Murata, Kosaku, c/o Kyoto University**
**Research Institute for Food Science,**
**Goka-sho**
**Uji-shi, Kyoto-fu (JP)**
Inventor: **Kimura, Akira, 81 Kami-wakamiya-cho**
**Rokujo-agaru,**
**Wakamiya-dori,**
**Shimogyo-ku**
**Kyoto-shi, Kyoto-fu (JP)**

(74) Representative: **Myerscough, Philip Boyd et al**
**J.A.KEMP & CO.**
**14, South Square**
**Gray's Inn**
**London WC1R 5LX (GB)**

## Description

### FIELD OF THE INVENTION

This invention relates to a novel recombinant DNA and a method for the production of a physiologically active substance such as, for example, glutathione.

### BACKGROUND OF THE INVENTION

A physiologically active substance such as, for example, glutathione has been heretofore produced by organic synthesis, extraction from cultured cell bodies (particularly yeast) of microorganisms, and a method which effects the production with a colon bacillus having the activities of the two enzymes, i.e. a $\gamma$ -glutamyl-L-cysteine synthetase (hereinafter referred to briefly as "GSH-I") and a glutathione synthetase (hereinafter referred to briefly as "GSH-II"), concerning synthesis of glutathione improved by genic recombination, for example.

The glutathione is a tripeptide consisting of glutamic acid, cysteine, and glycine. This is an important compound frequency used as a medicine for a great variety of hepatic diseases or as a biochemical reagent.

The two former methods do not necessarily deserve to be called advantageous measures because of long duration and intricacy of their reaction steps and on account of operational complexity and low intracellular content of their products. The latter method has a disadvantage that the production of glutathione is restricted by low GSH-I activity. In the circumstances, the desirability of developings method for producing such physiologically active substances as glutathione with prominent efficiency has been finding growing recognition.

### SUMMARY OF THE INVENTION

The present inventors formerly made various studies in search of a solution for the problem mentioned above and consequently acquired a knowledge that glutathione is produced in a higher yield as compared with the conventional method by preparing a recombinant DNA having inserted in a bacteriophage vector DNA a DNA containing a gene coding for GSH-I (hereinafter referred to briefly as "GSH-I gene") and a DNA containing a gene coding for GSH-II (hereinafter referred to briefly as "GSH-II gene"), culturing in a culture medium a glutathione-producing strain of genus Escherichia having the recombinant incorporated therein, and collecting glutathione from the resultant culture broth or inducing a contact catalysis between the cultured cell bodies of the microorganism and/or processed cell bodies of the microorganism on one part and glutamic acid, cysteine, glycine, adenosine-5′-triphosphoric acid, and magnesium ion on the other part. The invention issuing from this knowledge has been filed for patent in Japan (Japanese Patent Application SHO 63(1988)-52,659)and is disclosed in EP-A-0 107 400.

The present inventors continued studies and consequently acquired a knowledge that a physiologically active substance (glutathione) is produced in a high yield by preparing a recombinant DNA having inserted in a vector DNA containing a DNA containing a gene coding for an enzyme capable of synthesizing a physiologically active substance by conversion of ATP to ADP (comprising a DNA containing a gene coding for GSH-I and a DNA containing a gene coding for GSH-II, for example, where the physiologically active substance is glutathione) and a DNA containing a gene coding for acetate kinase (hereinafter referred to briefly as "ACK gene"), culturing in a culture medium a physiologically active substance (such as, for example, glutathione)-producing microorganism of genus Escherichia having the recombinant incorporated therein, and collecting the physiologically active substance (such as, for example, glutathione) from the resultant culture broth and that glutathione is produced in a much higher yield than the method just described by culturing in a culture medium a glutathione-producing microorganism of genus Escherichia containing a recombinant DNA having inserted in a vector DNA a DNA containing a gene coding for a $\gamma$ -glutamyl-L-cysteine synthetase, a DNA containing a gene coding for a glutathione synthetase, and a DNA containing a gene coding for acetate kinase and inducing a contact catalysis between the resultant cultured cell bodies of microorganism and/or processed cell bodies of microorganism on one part and glutamic acid, cysteine, glycine, adenosine-5′-triphosphoric acid, acetylphosphoric acid, and magnesium ion on the other part. The present invention has been perfected as the result.

The acetate kinase is an enzyme which catalyzes the reaction represented by the following reaction formula:

$$\text{ADP} + \text{Acetylphosphoric acid} \xrightleftharpoons{\text{Acetate kinase}} \text{ATP} + \text{Acetic acid}$$

The acetate kinase is highly useful as for the production of ATP. Cloning of the acetate kinase gene from E.coli is disclosed in Journal of Bacteriology 171(1),577-580(1989).

Specifically, this invention is directed to a novel recombinant DNA, characterized by having inserted in a vector DNA a DNA containing a gene coding for an enzyme capable of synthesizing a physiologically active substance by conversion of ATP to ADP (comprising a DNA containing a gene for coding a $\gamma$ -glutamyl-L-cysteine synthetase and a DNA containing a gene coding for a glutathione synthetase, for example) and a DNA containing a gene coding for acetate kinase. This invention is further directed to a method for the production of a physiologically active substance, characterized by culturing in a culture medium a physiologically active substance (such as, for example, glutathione or creatine phosphoric acid)-producing microorganism of genus Escherichia containing a recombinant DNA having inserted in a vector DNA a DNA containing a gene coding for an enzyme capable of synthesizing a physiologically active substance by conversion of ATP to ADP (comprising a DNA containing a gene coding for a $\gamma$ -glutamyl-L-cysteine synthetase and a DNA containing a gene coding for a glutathione synthetase, for example) and a DNA containing a gene coding for acetate kinase and collecting the physiologically active substance (such as, for example, glutathione or creatine phosphoric acid) from the resultant culture broth. This invention is also directed to a method for the production of glutathione, characterized by culturing in a culture medium a glutathione-producing microorganism of genus Escherichia containing a recombinant DNA having inserted in a vector DNA a DNA containing a gene coding for a $\gamma$ -glutamyl-L-cysteine synthetase, a DNA containing a gene coding for a glutathione synthetase, and a DNA containing a gene coding for acetate kinase and inducing a contact catalysis between the cultured cell bodies of the microorganism and/or processed cell bodies of the microorganism on one part and glutamic acid, cysteine, glycine, adenosine-5′-triphosphoric acid, acetylphosphoric acid, and magnesium ion on the other part.

As described above, the present invention, by obtaining a recombinant DNA having inserted in a bacteriophage vector DNA a DNA containing a gene coding for an enzyme capable of synthesizing a physiological substance by conversion of ATP to ADP (comprising a DNA containing a GSH-I gene and a DNA containing a GSH-II gene in the case of glutathione, for example) and a DNA containing an ACK gene, culturing in a culture medium a microorganism of genus Escherichia incorporating therein the recombinant DNA and consequently possessing an ability to produce a physiologically active substance (such as, for example, glutathione) thereby enhancing the amount of acetate kinase to be produced, enables the physiologically active substance (such as, for example, glutathione) to be produced from the culture broth in a high yield. Further, this invention, by culturing in a culture medium a microorganism of genus Escherichia containing a recombinant DNA having inserted in a vector DNA a DNA containing a gene coding for an enzyme for the synthesis of $\gamma$ -glutamyl-L-cysteine, a DNA containing a gene for coding an enzyme for the synthesis of glutathione, and a DNA containing a gene coding for acetate kinase and possessing an ability to produce glutathione and then inducing contact catalysis between the cell bodies of the microorganism resulting from the culture and/or processed cell bodies on one part and glutamic acid, cysteine, glycine, adenosine-5′-triphosphoric acid, acetyl phosphoric acid, and magnesium ion, attains production of glutathione in a high yield with low ATP content as compared with the conventional method. Thus, the present invention is highly useful.

BRIEF DESCRIPTION OF THE DRAWING

Fig. 1 is a diagram showing a restriction enzyme cleavage map of a recombinant bacteriophage 501GI-(lacP)-2ACK1GII (lacP)1 DNA.

DETAILED DESCRIPTION OF THE INVENTION

The vector to be used in this invention may be of any kind. It may be bacteriophage vector or plasmid vector, for example. The genes which possess the ability to code for an enzyme capable of synthesizing a physiological substance by conversion of ATP to ADP and which are usable in this invention include those which code $\gamma$ -glutamyl-L-cysteine synthetase, glutathione synthetase, creatine kinase, glycerol kinase, glucokinase, glutamine synthetase, and pyruvate kinase capable of synthesizing a physiologically pyruvic

acid, for example.

As means for inserting a DNA containing a gene coding for an enzyme capable of synthesizing a physiologically active substance by conversion of ATP to ADP, such as a DNA containing a GSH-I gene, a DNA containing a GSH-II gene, and a DNA containing an ACK gene, into a vector DNA and consequently incorporating the DNA into a host cell, the following methods may be cited. The first method accomplishes this incorporation by connecting a DNA containing a GSH-I gene, a DNA containing a GSH-II gene, a DNA containing an ACK gene to one and the same vector DNA and incorporating them in a strain originating in a colon bacillus. The second method comprises connecting a DNA containing a GSH-I gene, a DNA containing a GSH-II gene, and a DNA containing an ACK gene one each to three different vector DNA's, or connecting a DNA containing a GSH-I gene and a DNA containing an ACK gene to one and the same vector DNA and a DNA containing a GSH-II gene to a separate vector DNA, or connecting a DNA containing a GSH-I gene and a DNA containing a GSH-II gene to one and the same vector DNA and a DNA containing an ACK gene to a separate vector DNA, or connecting a DNA containing a GSH-II gene and a DNA containing and ACK gene to one and the same vector DNA and a DNA containing a GSH-I gene to a separate vector DNA. The third method comprises connecting a DNA containing a GSH-I gene, a DNA containing a GSH-II gene, and a DNA containing an ACK gene to one and the same vector DNA and incorporating them in a strain originating in colon bacillus and thereafter additionally incorporating in the strain a DNA having any of the genes mentioned above connected to a DNA vector.

Now, preparation of a recombinant DNA which has a DNA containing a GSH-I gene, a DNA containing a GSH-II gene, and a DNA containing an ACK gene connected onto one and the same phage DNA by the recombination of a phage DNA between a phage DNA having connected thereto a DNA containing a GSH-I gene and a phage DNA having connected thereto a DNA containing a GSH-II gene and a DNA containing an ACK gene by the use of a bacteriophage vector will be described. For the purpose of the connection to the vector DNA, any restriction enzyme which has no recognition site in the structure and promoter region of the DNA containing a GSH-I gene, and the DNA containing a GSH-II gene and an ACK gene may suffice. For example, the KpnI may be cited for the DNA containing a GSH-I gene under the control of a lac promoter and the EcoRI sectioned part for the DNA containing a GSH-II gene and the DNA containing an ACK gene.

First, the preparation of a recombinant DNA having inserted in a bacteriophage vector DNA a DNA restructured so as to have a DNA containing a GSH-I gene such as, for example, the aforementioned DNA originating in colon bacillus expressed with a strong promoter such as, for example, a lac promoter will be described.

The bacteriophage vector DNA may be of any form so long as it possesses an endonuclease sectioned part only in the genetic information part of the coating protein. The bacteriophage EN501S-Tc DNA may be cited, for example.

The bacteriophage EN501S-Tc DNA mentioned above can be prepared by the following method. First, a bacteriophage $\lambda cI_{857}$1121 DNA is obtained by faithfully following a working example cited in the specification of Japanese Patent Application Disclosure SHO 58(1983)-212,781 and a marker is attached to the upstream region or downstream region with an anaphasic promoter for facilitating the separation of the bacteriophage DNA. Any marker may suffice for the labeling. A drug-resistant marker may be cited, for example.

The impartation of a drug-resistant marker is attained simply by inserting a tetracycline-resistant gene (hereinafter referred to simply as "$Tc^r$") into a restriction enzyme EcoRI sectioned part presenting only in the downstream with an anaphasic promoter. Owing to the ensuant tetracycline-resistance, this bacteriophage DNA can be easily separated.

The tetracycline-resistant bacteriophage DNA such as, for example, EN1121Tc/xb, can be prepared as follows.

A DNA digestant is obtained by having plasmid pKN206 DNA [obtained by the method disclosed in T. Masuda et al, "Agri. Biol. Chem.," vol. 51, pages 271-278 (1986)] digested with a restriction enzyme Pvu II, for example.

By mixing this DNA digestant with an EcoRI linker and then causing a DNA ligase originating in T4 bacteriophage (produced by Boehringer Mannheim-Yamanouchi K.K.) to react on and link to the resultant mixture, there can be prepared a recombinant plasmid pKN306 DNA possessing an EcoRI sectioned part. The transformation with the recombinant plasmid pKN306 DNA can be effected by the treatment with calcium chloride disclosed in Molec. Gen. Genet., vol. 124, pages 1 to 10 (1973). As an example of the host microorganism to be used in the transformation, Colon Bacillus (procured from Max-Planck-Institute, Heidelberg, West Germany) may be cited.

From the transforming strain obtained as described above, purified plasmid DNA pKN306 can be obtained by the treatment of cesium chloride-ethidium bromide density gradient supercentrifugal separation as generally practised. A DNA segment containing a tetracycline-resistant gene is obtained by causing an EcoRI to react upon this plasmid pKN306 DNA. This DNA segment can be isolated by the treatment of agarose electrophoresis disclosed in "Methods in Enzymology," vol. 68, pages 176 to 182 (1979), for example.

By adding $\lambda cI_{857}$1121 DNA sectioned with a restriction enzyme EcoRI, for example and a 5′-pAATTGTCTAGA linker containing a part capable of discerning XbaI (produced by a DNA synthesizing machine, PLUSI, made by Beckman), for example, to the DNA segment mentioned above and linking them by the treatment with a T4 DNA ligase (produced by Boehringer Mannheim-Yamanouchi K.K.), there can be bred a bacteriophage $\lambda cI_{857}$1121-Tc/xb containing a tetracycline-resistant gene.

Then, from this bacteriophage $\lambda cI_{857}$1121-Tc/xb, the DNA is obtained in a refined form by the method proposed by T. Maniatis et al in "Molecular Cloning," pages 76 to 85 (1982) as generally practised.

Now, the preparation of a bacteriophage DNA possessing an endonuclease sectioned part only in the part of genetic information for the synthesis of a coating protein of bacteriophage DNA and having a drug-resistant marker attached to the downstream of the anaphasic promoter will be described below.

The drug-resistant marker may be of any kind. It may be a bacteriophage DNA labeled with a $Tc^r$ marker, for example. Further, the endonuclease sectioned part capable of exclusively discerning the genetic information part for the synthesis of a coating protein may be of any kind. It may be an EcoRI sectioned part or a KpnI sectioned part, for example.

The bacteriophage DNA possessing an endonuclease sectioned part only in the part of the genetic information for the synthesis of a coating protein can be obtained by faithfully following the method disclosed in Japanese patent Publication SHO 61(1986)-37,915. For example, a digested substance is obtained by causing a restriction enzyme such as, for example, EcoRI to react upon a bacteriophage 12-2 DNA obtained by faithfully following the method of Example (6) cited in the specification of Japanese Patent Application Disclosure SHO 58(1983)-212,781 and a bacteriophage λgtWES.B DNA (produced by Bethesda Research Laboratory), this digested substance is subjected to a connecting reaction by the use of a T4 DNA ligase, and the resultant DNA is transduced in a colon bacillus 1100, for example. The transduction can be effected by the method of calcium chloride treatment disclosed in D. M. Morrison, "Methods in Enzymology," vol. 68, pages 326 to 331 (1979), for example. By this method, a bacteriophage 12-2WE which forms a plaque on a colon bacillus such as, for example, the strain colon bacillus 1100 can be obtained. By infecting this bacteriophage 12-2WE and a bacteriophage $\phi$80 [obtained from E.coli W3110 ($\phi$80) (ATCC 31277) by the method described in Example (1) cited in the specification of Japanese Patent Application Disclosure SHO 58(1983)-212,781] with colon bacillus in entirely the same method as described in Example (2) cited in the specification of Japanese Patent Application Disclosure SHO 58(1983)-212,781, a bacteriophage 12-2-300 DNA capable of forming a plaque can be obtained.

Then, a bacteriophage DNA possessing an endonuclease sectioned part only in the part of genetic information of a coating protein and having a drug-resistant marker attached to the downstream area of the anaphasic promoter can be obtained by recombining the recombinant bacteriophage DNA possessing an endonuclease sectioned part only in the genetic information part of a coating protein and a bacteriophage DNA having a drug-resistant marker attached to the downstream area of the anaphasic promoter by the use of XhoI, for example.

By causing a restriction enzyme, XhoI (produced by Takara Shuzo Co., Ltd.) to react on the bacteriophage 12-2-300 DNA obtained as described above and a bacteriophage $\lambda cI_{857}$1121-Tc/xb thereby producing a digested substance as generally practised, subjecting this digested substance to a connecting reaction using a T4 DNA ligase, then transforming a colon bacillus treated with calcium chloride by the aforementioned method of D. M. Morrison such as, for example, a colon bacillus QD5003 strain (secured from Kyushu University) by the use of the product of the connection reaction, there can be obtained a bacteriophage EN501S-Tc which forms a plaque on the strain mentioned above.

In the manner described above, the target bacteriophage DNA which possesses an endonuclease sectioned part only in the part of genetic information for the coating protein and has a drug-resistant marker attached to the downstream area of the anaphasic promoter can be obtained.

The DNA containing the GSH-I gene originating in colon bacillus can be obtained as a recombinant plasmid pBR325-gshI-II DNA by faithfully following the procedure described in the specification of Japanese Patent Application Disclosure SHO 59(1984)-192,088.

The promoter for expressing the GSH-I may be of any type. It is desired to be lac promoter, for example.

A case using the lac promoter will be cited below.

A digested DNA is obtained by digesting a recombinant plasmid pBR325-gshI-II DNA by the action of a restriction enzyme PstI, for example. A recombinant pBR322-gshI DNA can be obtained by causing T4 DNA ligase to react on the mixture of the aforementioned digested DNA with a plasmid DNA sectioned with a restriction enzyme PstI such as, for example, a pBR322 DNA.

Then, a digested DNA is obtained by digesting the recombinant plasmid pBR322-gshI DNA by the action of a restriction enzyme StuI, for example. By mixing this digested DNA with a PstI linker and causing a T4 DNA ligase, for example, to react upon the resultant mixture, there can be obtained a recombinant plasmid having PstI sectioned parts one each on the opposite outer sides of GSH-I gene on the recombinant plasmid pBR322-gshI DNA.

From a transformed strain obtained by transforming a colon bacillus such as, for example, E.coli 1100, by the conventional method such as, for example the treatment with calcium chloride using the recombinant plasmid obtained as described above, a purified recombinant plasmid DNA is obtained by the conventional method such as, for example, the method of cesium chloride density gradient ultracentrifugation.

By mixing this purified recombinant plasmid DNA with a plasmid vector such as, for example, pUC19, digesting the resultant mixture by the action of a restriction enzyme PstI, for example, and then causing T4 DNA ligase, for example, to react upon the digested DNA, there is obtained a recombinant plasmid possessing a GSH-I gene on the plasmid pUC19 DNA.

Then, a recombinant plasmid having a plurality of GSH-I genes incorporated therein is produced.

First, a plasmid vector DNA possessing sectioned parts of KpnI-SpeI-StuI-SacI in the order mentioned on the plasmid vector DNA is obtained by digesting a plasmid vector DNA such as, for example, pBR322 DNA, by the action of restriction enzymes AatII and BamHI, adding a synthetic DNA possessing sectioned parts KpnI, SpeI, StuI, and SacI in the order mentioned, for example, on the plasmid vector DNA to the digested DNA, and causing a T4 DNA ligase to act upon the resultant mixture.

Then, a recombinant plasmid which has an exogenous strong promoter such as, for example, lac promoter deposited on the upstream of the GSH-I gene and possesses the sectioned parts KpnI-SpeI-XbaI-KbaI on the opposite outer sides thereof is obtained by digesting the plasmid vector DNA obtained as described above by the action of restriction enzymes StuI and SacI, for example, thereby producing a digested DNA, digesting the recombinant plasmid possessing the GSH-I gene on the plasmid pUC19 DNA obtained as described above by the action of restriction enzymes PvuII and SacI, mixing the resultant digested DNA with the aforementioned digested DNA, and causing a T4 DNA ligase, for example, to act upon the resultant mixture.

A digested DNA is obtained by causing restriction enzymes XbaI and SpeI (both produced by Boehringer Manheim-Yamanouchi K.K.), for example to act upon the recombinant plasmid obtained as described above.

Further by digesting the same plasmid by the action of a restriction enzyme SpeI, for example, mixing the resultant digested DNA with the digested DNA mentioned above, and causing a T4 DNA ligase to act upon the resultant mixture, there is obtained a recombinant plasmid DNA possessing a plurality (two, for example) of GSH-I genes prevented from being expressed by the promoter of the plasmid itself or an exogenous promoter such as, for example, a lac promoter and having sectioned parts of KpnI one each on the opposite outer sides thereof.

Various recombinant DNA's are obtained by mixing the recombinant plasmid DNA obtained as described above with the aftermentioned bacteriophage vector EN501STc DNA, digesting the resultant mixture by the action of a restriction enzyme KpnI, for example, and causing a T4 DNA ligase to act upon the resultant digested DNA.

The separation of a particular recombinant DNA from the mixture of various recombinant DNA's obtained as described above is attained by the following method.

First, by causing a colon bacillus lysogenized in advance with a temperate phage possessing the same cohesive ends and the same immunity as the phage used in the preparation of the recombination DNA to be infected with a large amount of a temperate phage possessing the same cohesive ends and the same immunity and no attachment side (the site at which the recombination of the host bacterium with the chromosome occurs during the lysogenization) for the host chromosome, mixing the resultant infected colon bacillus with the recombinant DNA, and allowing the resultant mixture to stand at a temperature in the range of 20° to 40°C, the incorporation of the recombinant DNA in the cell body of the host bacterium can be accomplished (Helper Method).

Otherwise, the incorporation of the recombinant DNA in the cell body of the host bacterium may be effected by the calcium chloride method, for example.

Alternatively, the incorporation of the recombination DNA in the cell body of the host bacterium may be attained by enclosing the various recombinant DNA's obtained as described above with the coating protein

of λ bacteriophage by the vitro packaging method [disclosed in "Methods in Enzymology," vol. 68, pages 281 to 298, 1979, Academic Press] thereby preparing bacteriophage particles, and infecting the bacteriophage particles with the host bacterium.

The host bacterium to be used in this case may be of any type so long as it belongs to the general E.coli K12 strain. Such colon bacilli as AB1157 (E.coli Genetic Stock Center, Yale University, USA), W3110 (ATCC27325), W3350 (ATCC27020), and 1100 (Max-Planck-Institute, West Germany), for example, may well be called particularly desirable.

By screening a microorganism attaining growth on a culture medium containing tetracycline (hereinafter referred to briefly as "Tc") from the aforementioned strains, there can be obtained a GSH-I-producing microorganism of genus Escherichia incorporating a recombinant DNA having inserted in the bacteriophage DNA a DNA containing a GSH-I gene. By the method described in "Science," vol. 202, page 1279 (1978), a purified recombinant DNA can be obtained.

The number of GSH-I gene-containing DNA's to be inserted in the bacteriophage vector DNA is required to be at least 1 and desired to be in the range of 2 to 8.

Now, the preparation of a recombinant DNA having inserted in a bacteriophage vector DNA a DNA containing a GSH-II gene and an ACK gene such as, for example, the aforementioned DNA originating in a colon bacillus will be described below.

The DNA containing the GSH-II gene originating in the colon bacillus can be obtained as a recombinant plasmid pBR322-gsHII DNA by faithfully following the procedure disclosed in the specification of Japanese Patent Application Disclosure SHO 60(1985)-180,592, for example.

Then, the plasmid pBR322-gsHII DNA is digested by the action of restriction enzymes BamHI and HindIII, for example, to obtain a digested DNA. The isolation of the digested DNA segment is easily effected by separating the product of the treatment with the enzymes BamHI and HindIII by the treatment of agarose gel electrophoresis and then extracting segment from the gel ["Methods in Enzymology," 68, 176 to 182 (1979)]. The isolated DNA segment is mixed with a plasmid pBR322 DNA treated with BamHI and HindIII, for example. The resultant mixture is treated with a T4 DNA ligase, for example, and then used in transforming a strain of colon bacillus rendered competent by the treatment with calcium such as, for example, a strain 1100. The strain possessing the target plasmid DNA is easily selected as a strain attaining growth on a T-Y culture medium containing Ap in a concentration of 20 μg/ml and no growth on a T-Y culture medium containing Tc in a concentration of 20 μg/ml.

Then, by digesting the recombinant plasmid DNA by the treatment with BamHI thereby producing a digested DNA segment, treating the digested DNA by following the method disclosed in "Molecular Cloning", pages 113 and 114, Cold Spring Harbor Laboratory (1982) thereby smoothening the digested DNA ends, mixing the resultant DNA of smoothened ends with EcoRI linker, and causing a T4 DNA ligase to act upon the resultant mixture, to obtain a plasmid DNA.

Now, the recombinant plasmid DNA obtained as described above is treated with DraII and EcoRI, the digested DNA segment is separated by means of agarose gel electrophoresis, and the separated DNA segment is mixed with a plasmid pUC19 treated with SmaI and EcoRI. The resultant mixture is treated with a T4 DNA ligase and then introduced in a strain of colon bacillus rendered competent by the treatment with calcium such as, for example E.coli JM101 strain. The strain possessing the target plasmid DNA can be easily selected as a strain attaining growth on a T-Y culture medium containing Ap in a concentration of 20 μg/ml and forming a white colony on a T-Y culture medium containing 5-bromo-4-chloro-3-indolyl-β-D-galactopyranoside and isopropyl-β-D-thiogalactopyranoside. Thus is obtained a recombinant plasmid DNA which possesses sectioned parts of PvuII one each on the opposite outer sides of the GSH-II gene expressed by the lac promoter.

Now, the preparation of a plasmid DNA having an ACK gene introduced inside the sectioned part of EcoRI of the plasmid DNA obtained as described above will be described.

The DNA containing an ACK gene can be isolated as a recombinant plasmid pAK122 DNA from the colon bacillus JM 101 (pAK122) (FERM BP-1534), for example.

This recombinant plasmid pAK122 DNA is treated with PstI and EcoRI, the resultant digested DNA segment is separated by means of agarose gel electrophoresis, the separated DNA segment is mixed with a plasmid DNA treated with PstI and EcoRI such as, for example, pBR322, and the resultant mixture is subjected to a connecting reaction by the action of a T4 DNA ligase, for example. The product of the connection reaction is used in transforming a strain of colon bacillus rendered competent by the reaction with calcium such as, for example E.coli 1100 strain. The strain containing the target plasmid can be easily selected as a strain attaining growth on a T-Y culture medium containing Ap in a concentration of 20 μg/ml. Thus, the recombinant DNA possessing an ACK gene on the plasmid pBR322 DNA can be obtained.

Then, the plasmid DNA possessing inside the aforementioned sectioned part of PvuII the GSH-II gene developed by the lac promoter is treated with PvuII, the resultant digested DNA segment is separated by means of agarose gel electrophoresis, the separated digested DNA segment is mixed with what is obtained by treating the recombinant DNA containing the ACK gene on the pBR322 obtained as described above with HpaI, and the resultant mixture is subjected to a connecting reaction by the use of a T4 DNA ligase, for example. The product of the connecting reaction is introduced into a strain of colon bacillus rendered competent by the treatment with calcium such as, for example, E.coli 1100 strain. The target recombinant plasmid DNA is detected as a recombinant plasmid DNA larger than the plasmid DNA having only an ACK gene incorporated therein in the plasmid DNA isolated from the strain attaining growth on a T-Y culture medium containing Ap in a concentration of 20 µg/ml. Thus, the recombinant plasmid DNA containing ACK genes and GSH-II genes developed by the lac promoter one each on the opposite inner sides of the sectioned part of EcoRI can be prepared.

The bacteriophage vector DNA may be of any type. The bacteriophage $\lambda cI_{857}1121S\Delta RzKm$ DNA described in Example 5 [7] herein below may be cited, for example.

Then, various recombinant DNA's are obtained by mixing the recombinant plasmid DNA obtained as described above with the bacteriophage vector $\lambda cI_{857}1121S\Delta RzKm$ DNA, digesting the resultant mixture by the action of a restriction enzyme EcoRI, and effecting connection of the DNA's by the action of a T4 DNA ligase.

The collection of the target recombinant DNA from the mixture of various recombinant DNA's obtained as described above is accomplished by the following method.

First, by causing a colon bacillus lysogenized in advance with a temperate phage possessing the same cohesive ends and the same immunity as the phage used in the preparation of the recombinant DNA to be infected with a large amount of a temperate phage possessing the same cohesive ends and the same immunity and no attachment site (the site at which the recombination with the chromosome of the host bacterium occurs during the lysogenization), mixing the resultant infected colon bacillus with the recombinant DNA, and allowing the resultant mixture to stand at a temperature in the range of 20° to 40°C, the incorporation of the recombinant DNA in the cell body of the host becterium can be accomplished (helper method).

The incorporation of the recombinant DNA in the cell body of the host bacterium can be otherwise attained by the calcium chloride method instead of the helper method.

Alternatively, the incorporation of the recombinant DNA in the cell body of the host bacterium can be accomplished by enclosing the various recombinant DNA's with the coating protein of a λ bacteriophage by the in vitro packaging method [disclosed in "Methods in Enzymology," vol. 68, pages 281 to 298, 1979, Academic Press], thereby preparing bacteriophage particles, and then infecting the bacteriophage particles with the host bacterium.

The host bacterium to be used in this case may be any colon bacillus so long as it belongs the general E.coli K12 strain. For example, such colong bacilli as AB1157 (E.coli Genetic Stock Center, Yale University, USA), W3110 (ATCC27325), W3350 (ATCC27020), and 1100 (Max-Planck-Institute, West Germany) may well be called particularly suitable.

By screening a microorganism attaining growth on a T-Y culture medium containing Kanamycin (hereinafter referred to briefly as "Km") in a concentration of 10 µg/ml and possessing an ability to produce GSH-II and ACK from the strain mentioned above, there can be obtained a microorganism of genus Escherichia containing a recombinant DNA having inserted in a bacteriophage vetor DNA a DNA containing a GSH-II gene and an ACK gene and possessing an ability to produce GSH-II and ACK.

The collection of the recombinant DNA in a purified state from the resultant microorganism is accomplished, for example, by thermally inducing culture of the lysogen retaining the recombinant DNA at a temperature in the range of 40° to 45°C, preferably 42° to 43°C, for a period exceeding 10 minutes, preferably falling in the range of 15 to 30 minutes, then shaking the cultured lysogen in the presence of chloroform, for example, at a temperature in the range of 0° to 40° C for a period in the range of 10 minutes to 1 hour thereby effecting lysis of the cell body of the host bacterium and consequent discharge of bacteriophage in the culture medium, subjecting the culture broth to centrifugation at a rate in the range of 7,000 to 20,000 r.p.m. for a period in the range of 10 to 30 minutes thereby removing cell remains, purifying the residual bacteriophage by ultracentrifugation at a rate in the range of 20,000 to 40,000 r.p.m. for a period in the range of 1 to 3 hours, and subjecting the purified bacteriophage to molecular cloning by the method disclosed in Cold Spring Harbor Laboratory, pages 76 to 85 (1982), for example.

The number of DNA's containing GSH-II gene and intended for insertion in the bacteriophage vector DNA is required to be at least one and desired to be one or two and the number of DNA's containing an ACK gene is required to be at least one and desired to be one or two.

Various recombinant DNA's are obtained by mixing the ensuant recombinant DNA having inserted in a bacteriophage vector DNA a DNA containing a GSH-II gene and an ACK gene with a recombinant DNA having inserted in a bacteriophage vector DNA a DNA containing a GSH-I gene developed by a strong promoter such as, for example, a lac promoter, digesting the resultant mixture by the action of a restriction enzyme NheI, for example, and connecting the component recombinant DNA's of the digested mixture by the action of a T4 DNA ligase, for example.

Thereafter, by following the aforementioned procedure for the preparation of a microorganism of genus Escherichia and a recombinant DNA containing a recombinant DNA having inserted in a bacteriophage vector DNA a DNA containing a GSH-II gene and an ACK gene and possessing an ability to produce GSH-II and ACK, a microorganism of genus Escherichia containing a recombinant DNA having inserted in a bacteriophage vector DNA a DNA containing a GSH-I gene and a DNA containing a GSH-II gene and an ACK gene and possessing an ability to produce glutathione can be obtained. Further, the recombinant DNA can be obtained in purified form by following the aforementioned procedure for the purification of a recombinant DNA having inserted in a bacteriophage vector DNA a DNA containing a GSH-I gene.

Then, the microorganism of genus Escherichia containing a recombinant DNA having inserted in a bacteriophage vector DNA a DNA containing a GSH-I gene, a DNA containing a GSH-II gene, and a DNA containing an ACK gene and possessing an ability to produce glutathione is cultured under the conditions incapable of inducing self-replication of the recombinant DNA, namely at a temperature in the range of 30° to 35°C for a period in the range of 2 to 24 hours.

The culture medium to be used in this case is a culture medium generally employed for the culture of any ordinary microorganism. It is desired to be a T-Y culture medium, for example.

Subsequently, the culture mixture is subjected to heat induction (at a temperature in the range of 40° to 45°C), UV treatment, or addition of mitomycin to induce self-replication of the recombinant DNA and then left standing at a temperature enough to permit multiplication of the host bacterium.

At this time, the temperature of the culture mixture is not always required to be maintained at a level for inducing the self-replication of the recombinant DNA but may be kept at a suitably selected level in the range in which the host bacterium is allowed to multiply.

After the self-replication of the novel recombinant DNA is induced as described above, the culture is continued for a period in the range of 2 to 6 hours, for example, to obtain the culture aimed at.

Now, the production of glutathione will be described.

The glutathione in the cell bodies of colon bacillus can be extracted by collecting cell bodies from the culture broth, washing the cell bodies once with a 0.85% physiological saline solution, suspending the washed cell bodies in water, and heat treating the aqueous suspension in boiling water at 100°C for a period in the range of 1 to 10 minutes, preferably 1 minute. The glutathione can be produced by collecting the cell bodies after the culturing mentioned above, treating the collected cell bodies as described below, and allowing the treated cell bodies to react with glutamic acid, cysteine, glycine, magnesium ion, ATP, and acetylphosphoric acid. The products of such cell treatment include a product of the treatment of cell bodies with an organic solvent, a product of treatment thereof with a surfactant, a product of the attrition of cell bodies by supersonic wave, a cell-free extract obtained by centrifugation after the treatment with the supersonic wave, the cell bodies fixed on a suitable carrier, and an enzyme, for example. The organic solvents which are usable herein include acetone, toluene, and ether, for example. The surfactants which are usable herein include Triton® X100, dodecylsulfuric acid, and cetyltrimethyl ammonium bromide, for example. The carriers which are usable for the immobilization of the cell bodies or enzyme include polyacryl amide gel, arginic acid gel, photosetting resin, and DEAE-cellulose, and DEAE-Sephadex®, for example.

The treatment for the formation of glutathione can be carried out by keeping the enzyme-containing substance in contact with a reaction solution containing 5 to 160 mM of glutamic acid, 5 to 80 mM of cysteine, 5 to 100 mM of glycine, 1 to 30 mM of magnesium ion, 0.01 to 20 mM of ATP, and 200 to 0 mM of acetylphosphoric acid at a temperature in the range of 20° to 40°C [preferably at 37°C) at a pH in the range of 6 to 9 (preferably 7.5) for several hours.

The glutathione extracted or formed in the reaction solution as described above is easily isolated with a conventional column of an ion-exchange resin. The glutathione is isolated in crystalline form by adjusting the extract or the reaction solution with sulfuric acid to pH 3.0, passing the pH-adjusted solution through a bed of a cation-exchange resin such as, for example, Diaion® PK-228$H^+$, eluting the adsorbade with 0.5M ammonium hydroxide, adjusting the eluate with sulfuric acid to pH 4.5, passing the pH-adjusted eluate through a bed of an anion-exchange resin such as, for example, Deolite® A2 ($CH_2COO^-$ form), eluting the adsorbed glutathione with 0.5M sulfuric acid, and diluting the eluate with ethanol added to 50% of the total volume.

EXAMPLES

Now, the present invention will be described more specifically below with reference to working examples.

[1] Preparation of DNA of recombinant plasmid pGS1102

(1) Preparation of E.coli 1100 (pBR322-gshIp)

From E.coli RC 912 (FERM BP-337) retaining a recombinant plasmid pBR322-gshI•II DNA, the recombinant plasmid pBR322 gshI•II DNA (disclosed in Japanese Patent Publication HEI 1(1989)-42,672) was isolated by the method of T. Maniatis ["Molecular cloning," Cold Spring Harbor Laboratory, pages 86 to 96 [1982)], for example, to obtain 1 mg of the DNA in purified form.

A DNA segment was obtained by mixing 5 μg of the recombinant plasmid pBR322-gshI DNA with 5 units of PstI (produced by Boehringer Mannheim-Yamanouchi K.K.), heating the resultant mixture at 37° C for 1 hour thereby inducing digestion of the DNA, and subjecting the digested DNA to the conventional treatment of agarose gel electrophoresis. By cutting out the gel containing the DNA segment, placing it in a dialytic tube, and subjecting it to the treatment of electrophoresis thereby removing the DNA segment out of the gel, there was obtained 2 μg of a DNA segment gshIp containing the GSH-I gene.

Then, 1 μg of the plasmid pBR322 DNA and 5 units of PstI added thereto were heated at 37° C for 1 hour to digest the DNA. The digested DNA and 1 μg of the DNA segment gshIp added thereto were left reacting at 16° C for 12 hours to effect connection of the DNA's and produce a recombinant plasmid pBR322-gshI DNA retaining a GSH-I gene on the plasmid pBR322 DNA.

Then, E.coli 1100 (procured from Max-Planck-Institute, Heidelberg, West Germany) was transformed by the method disclosed in "J. Bacteriol.," vol. 119, pages 1072 to 1074 (1974) with the recombinant plasmid pBR322-gshI DNA obtained as described above. Consequently, there was obtained a transformed strain or E.coli 1100 (pBR322-gshI) which attained growth on a T-Y culture medium containing Ampicillin (hereinafter referred to briefly as "Ap") in a concentration of 2 μg/ml and attaining no growth on a T-Y culture medium containing tetracycline (hereinafter referred to briefly as "Tc") in a concentration of 20 μg/ml.

Subsequently, by isolating the recombinant plasmid pBR322-gshI DNA from the E.coli 1100 (pBR322-gshI) by the aforementioned method, there was obtained 1 mg of DNA.

A recombinant plasmid pBR322-gshIp DNA possessing sectioned parts PstI one each on the opposite sides of a gshI gene on a recombinant DNA pBR322-gshI was obtained by mixing 5 μg of the recombinant plasmid pBR322-gshI DNA with 5 units of StuI (produced by Boehringer Mannheim-Yamanouchi K.K.), heating the resultant mixture at 37° C for 1 hours digestion of the DNA, subjecting the digested DNA to the conventional treatment with phenol and the treatment for ethanol precipitation thereby producing a precipitate, dissolving the precipitate in 20 μl of water, treating the resultant solution by the method disclosed in "Molecular Cloning," pages 113-114, Cold Spring Harbor Laboratory (1982) using Klenow Fragment of E.coli DNA polymerase I (produced bY Takara Shuzo Co., Ltd.), and causing 1 unit of T4 DNA ligase (produced by Boehringer Mannheim-Yamanouchi K.K.) to act at 16°C for 16 hours upon the mixture of treated solution with 10 μg of PstI linker.

The aforementioned E.coli 1100 (procured from Max-Planck-Institute, Heidelberg, West Germany) treated in advance with calcium chloride was transformed by method disclosed in "J. Bacteriol," vol. 119, pages 1072 to 1074 (1974) using the recombinant plasmid pBR322-gshIp DNA. Consequently, there was obtained a transformed strain or E.coli 1100 (pBR322-gshIp) attaining growth on a T-Y culture medium containing Ampicillin (Ap) in a concentration of 2 μg/ml and no growth on a T-Y culture medium containing Tetracycline (Tc) in a concentration of 20 μg/ml.

(2) Preparation of E.coli JM101 (pUC19-gshIkp)

By isolating the recombinant plasmid pBR322-gshIp DNA from the E.coli 1100 (pBR322-gshIp) by the method described in (1) of Item [I], there was obtained 1 mg of DNA.

A DNA segment was obtained by mixing 5 μg of the recombinant plasmid pBR322-gshIp DNA with 5 units of PstI (produced by Boehringer Mannheim-Yamanouchi K.K.), heating the resultant mixture at 37° C for 1 hour for effecting digestion of the DNA, and subjecting the resultant digested DNA to the conventional method of agarose gel electrophoresis.

By cutting the gel containing the DNA segment, placing the gel in a dialytic tube, and subjecting the gel to the treatment of electrophoresis thereby removing the DNA segment out of the gel, there was obtained 2

μg of a DNA segment gshIpp containing a GSH-I gene.

Then, a recombinant plasmid pUC19-gshIkp DNA retaining the GSH-I gene on the plasmid pUC19 DNA was obtained by mixing 1 μg of the plasmid pUC19 DNA (produced by Takara Shuzo Co., Ltd.) with 5 units of PstI (produced by Boehringer Mannheim-Yamanouchi K.K.), heating the resultant mixture at 37 °C for 1 hour for effecting digestion of the DNA, combining the digested DNA with 1 μg of the DNA segment gshIpp obtained as described above, and allowing the resultant mixture and 1 unit of the T4 DNA ligase added thereto react at 16 °C for 12 hours for effecting connection of the DNA's.

Then, a transformed strain, i.e. E.coli JM101 (pUC19-gshIkp), attaining growth on a T-Y culture medium containing Ap in a concentration of 20 μg/ml and forming a white colony on a T-Y culture medium containing 5-bromo-4-chloro-3-indolyl-β-D-galactopyranoside, and isopropyl-β-thiogalactopyranoside was obtained by causing E.coli JM101 (produced from Takara Shuzo Co., Ltd.) treated in advance with calcium chloride to be transformed by the method described in (1) of Item [I] using the recombinant pUC19-gshIkp DNA obtained as described above.

(3) Preparation of plasmid vector PBR322L-Aab DNA

A plasmid pBR322L-Aab DNA possessing sectioned parts of ArtII, KpnI, SpeI, StuI, SacI, and BamHI on the plasmid DNA was obtained by mixing 1 μg of a plasmid pBR322 DNA (produced by Takara Shuzo Co., Ltd.) with 2 units of AatII (produced by Boehringer Mannheim-Yamanouchi K.K.) and 2 units of BamHI (produced Boehringer Mannheim-Yamanouchi K.K.), heating the resultant mixture at 37 °C for 1 hour to effect digestion of the DNA, subjecting the digested DNA to the conventional treatment with phenol and the treatment for ethanol precipitation thereby producing a precipitate, dissolving the precipitate in 17 μl of water, combining the aqueous solution with 0.3 μg each of a 26-base oligonucleotide of 5′CGGTACCACTAGTAGGCCTGAGCTCG3′ and a 34-base oligonucleotide of 5′GATCCGAGCTCAGGCCTACTAGTGGTACCGACGT3′ synthesized with a DNA synthesizing machine (produced by Beckman), and causing 1 unit of the T4 DNA ligase to react at 20 °C for 16 hours on the resultant mixture.

By causing the E.coli 1100 treated in advance with calcium chloride to be transformed by the method described in (1) of Item [I] using the plasmid DNA mentioned above, there was obtained a transformed strain, i.e. E.coli 1100 (pBR322L-AaB), attaining no growth on a T-Y culture medium containing tetracycline (Tc) in a concentration of 20 μg/ml and attaining growth on a T-Y culture medium containing an Ampicillin (Ap) in a concentration of 20 μg/ml.

By isolating the plasmid PBR322L-AaB by the method described in (1) of Item [I] from the E.coli 1100 (pBR322L-AaB) obtained as described above, there was obtained 1 mg of DNA.

(4) Preparation of E.coli 1100 (pGS1101)

By causing 2 μg of the recombinant plasmid pUC19-gshIkp DNA obtained as described in (2) of Item [I], 1 unit of PvuII (produced by Boehringer Mannheim-Yamanouchi K.K.), and 1 unit of SacI (produced by Boehringer Mannheim-Yamanouchi K.K.) to react with one another in 20 μl of a solution containing 10 mM Tris-HCl (pH 7.5)/10 mM $MgCl_2$/1 mM dithioerythritol/50 mM NaCl at 37 °C for 1 hour, subjecting the resultant gel electrophoresis thereby effecting separation of a DNA segment, cutting out the gel containing the DNA segment, placing this gel in a dialytic tube, subjecting it to the treatment of electrophoresis, removing the DNA segment out of the gel, there was obtained 0.6 μg of the DNA segment gshIps containing a GSH-I gene.

Then, by causing 2 μg of the plasmid pBR322L-AaB DNA obtained as described in (3) of Item [I] and 1 unit of StuI and 1 unit of SacI (produced by Boehringer Mannheim-Yamanouchi K.K.) to react with one another in 20 μl of a solution containing 10 mM Tris-HCl (pH 7.5)/10 mM $MgCl_2$/1 mM dithioerythritol at 37 °C for 1 hour, subjecting the resultant reaction solution to the conventional treatment with phenol and the treatment for ethanol precipitation thereby producing a precipitate, dissolving the precipitate in 20 μl of water, combining the resultant solution with 0.5 μg of the DNA segment gshIps obtained as described above and 1 unit of the T4 DNA ligase, and heating the resultant mixture at 16 °C for 12 hours for reaction a plasmid pGS1101 DNA is obtained.

Then, by causing E.coli 1100 treated in advance with calcium chloride to be transformed by the method described in (1) of Item [I] with the plasmid DNA, there was obtained a transformed strain, i.e. E.coli 1100 (pGS1101), attaining growth on a T-Y culture medium containing Ap in a concentration of 20 μg/ml.

(5) Preparation of E.coli 1100 (pGS1102)

By isolating the recombinant plasmid pGS1101 DNA by the method described in (1) of Item [I] from the E.coli 1100 (pGS1101), 0.9 mg of DNA was obtained in purified form.

By causing 6 μg of the recombinant plasmid pGS1101 DNA obtained as described above and 3 units of XbaI and 3 units of SpeI (both produced by Boehringer Mannheim-Yamanouchi K.K.) to react with one another in 60 μl of a solution containing 50 mM Tris-HC1 (pH 7.5)/10 mM $MgCl_2$/l mM dithioerythritol/100 mM NaCl at 37°C for 1 hour, subjecting the resultant reaction solution to the conventional treatment of agarose gel electrophoresis thereby effecting separation of the DNA segment, cutting out the gel containing the DNA segment, placing the gel in a dialytic tube, and subjecting it to the treatment of electrophoresis thereby removing the DNA segment out of the gel, there was obtained 2 μg of the DNA segment gshI(lac-P)xs containing the GSH-I gene.

Subsequently, 2.0 μg of various recombinant plasmid DNA's containing the recombinant plasmid pGS1102 DNA were obtained by causing 1 μg of the pGS1101 DNA and 1 unit of SpeI to react with each other in 20 μl of a solution containing 50 mM Tris-HCl (pH 7.5)/10 mM $MgCl_2$/l mM dithioerythritol/100 mM NaCl at 37°C for 1 hour, subjecting the resultant reaction solution to the conventional treatment with phenol and the treatment for ethanol precipitation thereby producing a precipitate, dissolving the precipitate in 20 μl of water, combining the aqueous solution of precipitate with 2 μg of the DNA segment gshI(lac-P)xs and 1 unit of the T4 DNA ligase, and heating the resultant mixture at 16°C for 12 hours for reaction.

By causing the E.coli 1100 treated in advance with calcium chloride to be transformed by the method described in (1) of Item [I] with the recombinant plasmid DNA, there was obtained a transformed strain, i.e. E.coli 1100 (pGS1102), attaining growth on a T-Y culture medium containing Ap in a concentration of 20 μg/ml.

The recombinant plasmid DNA was isolated by the method of alkali extraction described in "Nucleic Acids research," vol. 7, pages 1513 to 1523 from the transformed strain obtained as described above and then treated by agarose electrophoresis for separation into recombinant plasmid DNA fractions. In the separated fractions, the recombinant plasmid incorporating two GSH-I genes was found to be larger than the recombinant plasmid DNA incorporating one GSH-I gene. In other words, this was detected as a recombinant plasmid DNA having a small distance of movement in the agarose electrophoresis. Thus was obtained a recombinant plasmid pGS1102 DNA containing two GSH-I genes developed by lactose promoter one each on the inside of the sectioned part of KpnI.

(6) Isolation of recombinant plasmid pGS1102 DNA

From the E.coli 1100 (pGS1102), 0.7 mg of a recombinant plasmid pGS1102 DNA was obtained by the method described in (1) of Item [I].

[2] Breeding of bacteriophage EN501S-Tc

(1) Breeding of bacteriophage $\lambda cI_{857}1121$

By faithfully following the procedure of a working example cited in the specification of Japanese Patent Application Disclosure SHO 58(1983)-212,781, bacteriophage $\lambda cI_{857}1121$ [which has been deposited at Fermentation Research Institute, Agency of Industrial Science and Technology, as E.coli 1100 ($\lambda cI_{857}1121$) [FERM BP-133], namely a lysogen obtained by causing this phage to lysogenize E.coli 1100 (procured from Max-Plank-Institute, Heidelberg, West Germany) as generally practised was prepared. The DNA of bacteriophage $\lambda cI_{857}1121$ was obtained by the method of T. Maniatis ["Molecular Cloning," pages 76 to 85 (1982)], for example, from this bacteriophage $\lambda cI_{857}1121$.

(2) Breeding of bacteriophage $\lambda cI_{857}1121$-Tc/xb

By causing reaction of 1 μg of the DNA of bacteriophage $\lambda cI_{857}1121$ DNA obtained as described above with 10 units of EcoRI (produced by Takara Shuzo K.K.) in a 50 mM tris-hydrochloric acid buffer solution (containing 100 mM NaCl and 10 mM $MgSO_4$ and exhibiting pH 7.4) at 37°C for 1 hour and subjecting the ensuant reaction solution to the conventional treatments of phenol extraction and ethanol precipitation, there was obtained 0.8 μg of the EcoRI digestion product of the bacteriophage $\lambda cI_{857}1121$ DNA.

Separately, 0.8 μg of the PvuII digestion product of plasmid pKN206 DNA was obtained by causing reaction of 1 μg of plasmid pKN206 DNA [obtained by the method described in T. Masuda et al, "Agri. Biol.

Chem.," vol. 50, pages 271 to 278 (1986)] with 10 units of PvuII (produced by Takara Shuzo K.K.) in a 10 mM Tris-hydrochloric acid buffer solution (containing 50 mM NaCl, 10 mM $MgSO_4$, and 1 mM dithiothreitol and exhibiting pH 7.4) at 37°C for 2 hours and subjecting the resultant reaction solution to the conventional treatments of phenol extraction and ethanol precipitation.

Then, 0.8 μg of plasmid pKN306 DNA was obtained by causing reaction of 0.8 μg of the PvuII digestion product of plasmid pKN206 DNA with 1 μg of an EcoRI linker having the 5′ end phosphorylated (produced by New England Biolabs) and 1 unit of the T4 DNA ligase (produced by Boehringer Mannheim-Yamanocuhi K.K.) in a 66 mM Tris-hydrochloric acid buffer solution (containing 6.6 mM $MgCl_2$, 10 mM dithiothreitol, and 10 mM ATP and exhibiting pH 7.5) at 4°C for 20 hours.

E.coli DH 1 (pKN306) was obtained by causing E.coli DH1 (ATCC33849) treated in advance with calcium chloride to be transformed by the method of D. M. Morrison ["Methods in Enzymology," vol. 68, pages 326 to 331 (1979)] using the plasmid pKN306 DNA obtained as described above.

In entirely the same manner as described in (1) of Item [I], 1200 μg of plasmid pKN306 DNA was obtained.

By causing reaction of 10 μg of this DNA with 50 units of EcoRI (produced by Takara Shuzo Co., Ltd.) in a 50 mM Tris-hydrochloric acid buffer solution (containing 100 mM NaCl and 10 mM $MgSO_4$ and exhibiting pH 7.4) at 37°C for 2 hours and subjecting the resultant reaction solution to extraction by the method of R. C. A. Yang et al ["Methods in Enzymology," vol. 68, pages 176 to 182 (1979)], there was fractionally collected 3.5 μg of 2.3 Kb DNA segment containing a tetracycline-resistant gene.

By causing reaction of 1 μg of the 2.3 Kb DNA segment mentioned above with 1 μg of a synthesized 5′-pAATTGTCTAG linker (synthesized by the use of a PLUSI DNA synthesizing machine produced by Beckman), 1 μg of a bacteriophage $\lambda cI_{857}$1121 DNA sectioned with EcoRI, and 12 units of the T4 DNA ligase (produced by Boehringer Mannheim-Yamanocuhi Co., Ltd.) in a 66 mM Tris-hydrochloric acid buffer solution (containing 6.6 mM $MgSO_4$, 10 mM dithiothreitol, and 10 mM ATP and exhibiting pH 7.5) at 4°C for 20 hours and causing E.coli 1100 treated in advance with calcium chloride to be transformed by the method of D. M. Morrison mentioned above by using the DNA obtained as described above, there was obtained E.coli 1100 ($\lambda cI_{857}$1122-Tc/xb).

(3) Breeding of bacteriophage EN501S-Tc

By causing reaction of 2 μg of the bacteriophage $\lambda cI_{857}$1121-Tc/xb DNA obtained from E.coli 1100 ($\lambda cI_{857}$1122-Tc/xb) by the method of T. Maniatis et al ["Molecular Cloning," pages 76 to 85 (1982)] with 10 units of XhoI (produced by Takara Shuzo K.K.) in a 50 mM Tris-hydrochloric acid buffer solution (containing 100 mM NaCl and 10 mM $MgSO_4$ and exhibiting pH 7.4) at 37°C for 1 hour and subjecting the ensuant reaction solution to the conventional treatments of phenol extraction and ethanol precipitation, there was obtained 1.6 μg of the XhoI digested product of bacteriophage $\lambda cI_{857}$1121-Tc/xb DNA.

Separately, 1.6 μg of digested product was obtained by causing reaction of 1 μg of bacteriophage 12-2 DNA obtained by faithfully following the procedure described in Example (6) cited in the specification of Japanese Patent Application Disclosure SHO 58(1983)-212,781 with 1 μg of bacteriophage λgtWES.λB DNA (produced by Bethesda Research Laboratory) and 10 units of EcoRI (produced by Takara Shuzo Co, Ltd.) in a 50 mM Tris-hydrochloric acid buffer solution (containing 100 mM NaCl and 10 mM $MgSO_4$ and exhibiting pH 7.4) at 37°C for 1 hour and subjecting the resultant reaction solution to the conventional treatments of phenol extraction and ethanol precipitation.

By causing reaction of 1.6 μg of the digested product with 1 unit of the T4 DNA ligase in a 66 mM Tris-hydrochloric acid buffer solution (containing 6.6 mM $MgCl_2$, 10 mM dithiothreitol, and 10 mM ATP and exhibiting pH 7.5) at 4°C for 16 hours and then causing E.coli 1100 treated in advance with calcium chloride to be transformed by the method of D. M. Morrison mentioned above by the use of the DNA obtained above, there was obtained a bacteriophage 12-2WE forming a plaque on E.coli 1100.

A bacteriophage 12-2-300 forming a plaque was obtained by causing the bacteriophage 122WE obtained as described above and the bacteriophage $\phi$ (obtained from E.coli W3110 ($\phi$80) (ATCC31277) by the method described in example (1) cited in the specification of Japanese Patent Application Disclosure SHO 58(1983)-212,781) to infect E.coli 1100 thereby obtaining a bacteriolyzed solution, and causing this bacteriolyzed solution to infect E.coli W3110 ($\phi$80) (ATCC31277).

Then, 1.6 μg of the digested product was obtained by causing reaction of 2 μg of bacteriophage 12-2-300 DNA prepared from the bacteriophage 12-2-300 by the method of T. Maniatis mentioned above, for example with 20 units of XhoI in a 50 mM Tris-hydrochloric acid buffer solution (containing 100 mM NaCl and 10 mM $MgSO_4$ and exhibiting pH 7.4) at 37°C for 1 hour and subsequently subjecting the ensuant reaction solution to the conventional treatments of Phenol extraction and ethanol precipitation.

A bacteriophage EN501S-Tc forming a plaque on the E.coli QD5003 strain was obtained by causing reaction of 1 μg of the XhoI digestion product of the aforementioned bacteriophage 12-2-300 DNA and 1 μg of the XhoI digestion product of the aforementioned bacteriophage λcI$_{857}$1121-Tc/xb DNA with 1 unit of the T4 DNA ligase in a 66 mM Tris-hydrochloric acid buffer solution (containing 6.6 mM $MgCl_2$, 10 mM dithiothreitol, 10 mM ATP and exhibiting pH 7.50 at 16 °C for 16 hours and subsequently causing E.coli QD5003 (obtained from Kyushu University) treated in advance with calcium chloride to be transformed by the method of D. M. Morrison mentioned above by the use of the DNA obtained as described above.

[3] Production of recombinant bacteriophage λEN501S-GI (lacP)2 having GSH-I gene segment inserted under control of DNA configuration necessary for translation in sectioned part of endonuclease present in part of genetic information on production of coating protein for bacteriophage EN501S-Tc.

A connecting reaction was effected by mixing 10 μg of the bacteriophage EN501S-Tc DNA obtained in (3) of Item [2] with 30 μg of the recombinant plasmid pGS1102 DNA obtained in (6) of Item [1], adding the resultant mixture to 50 μl of a solution of the composition of 10 mM Tris-HCl (pH 7.5)/10 mM $MgCl_2$/1 mM dithiothreitol, further adding thereto 50 units of KpnI (produced by Boehringer Mannheim-Yamanouchi K.K.), heating the resultant mixture at 37 °C for 2 hours for reaction of the reactants, subjecting the reaction solution to the conventional treatments of phenol extraction and ethanol precipitation thereby producing a precipitate, adding this precipitate to 8 μl of a solution of the composition of 50 mM Tris-HCl (pH 7.4)/0.1 mM ATP, adding thereto 2 units of the T4 DNA ligase (produced by Boehringer Mannheim-Yamanouchi K.K.), and heating the resultant mixture at 4 °C for 18 hours thereby inducing reaction.

Lysogens were obtained by enclosing 10 μg of the DNA obtained as described above with the coating protein of λ bacteriophage by the in vitro packaging method [described in "Methods in Enzymology," vol. 68, pages 281 to 298, 1979, Academic Press] thereby preparing bacteriophage particles, allowing 50 μl of a solution of the phage particles and the aforementioned E.coli 1100 ($10^9$/ml, 0.5 μl) added thereto to stand at 30 ° C for 3 hours for the purpose of incubation, sprinkling the resultant incubated solution on a agar culture medium containing tetracycline (Tc) in a concentration of 0.15 μg/ml, culturing the microorganism at 32 ° C for 48 hours, and selecting a Tc-tolerant strain from the culture broth.

The lysogen was inoculated to a T-Y culture medium, shaken cultured at 32 °C for 16 hours. An inoculum, 0.5 ml of the broth, was inoculated to a 10-ml T-Y culture medium in a conical flask having an inner volume of 150 ml and, after the amount of Klett units increased to about 100, heated to 43 °C and shake at that temperature for 25 minutes, cooled to 32 °C and shaken at this temperature for about 3 hours.

A 1-ml portion of the culture broth consequently obtained was crushed with ultrasonic wave to obtain a cell extract. This cell extract was tested for enzymatic activity of GSH-I in accordance with the method described in "Journal of General Microbiology," vol. 128, pages 1047 to 1053 (1982), to select a strain of high activity. A 10-ml of the lysogen of high activity was inoculated to a T-Y culture medium, shaken cultured at 32 °C until the amount of Klett units increased to about 100, heated at 43 °C for 20 minutes, and further shaken cultured at 37 °C for 3 hours to produce a culture broth. From the culture broth, the DNA was extracted by using the same volume of a phenol-chloroform mixed solvent as the culture broth. The DNA thus obtained was subjected to ethanol precipitation to isolate the DNA.

The DNA thus obtained was analyzed for size of segment by dissolving the DNA in 20 μl of a solution of the composition of Tris-hydrochloric acid buffer solution (pH 7.5)/1mM EDTA, adding 4 μl of the resultant solution to 30 μl of a solution of the composition of 10 mM Tris-hydrochloric acid buffer solution (pH 7.5)/10 mM $MgCl_2$/1 mM dithioerythritol, further adding thereto 10 units of KpnI (produced by Boehringer Mannheim-Yamanouchi K.K.), allowing the resultant mixture to stand at 37 °C for 2 hours for the purpose of digestion, and subjecting the digestion product to the treatment of agarose electrophoresis.

As the result, the highly active lysogen of GSH-I was found to retain about 41 Kbp of a DNA segment originating in pGS1102.

As described above, the recombinant bacteriophage EN501S-GI(lacP)2 was produced by separating E.coli 1100 (EN501S-GI(lacP)2).

[4] Preparation of recombinant plasmid pAK323 DNA

(1) Preparation of E.coli 1100 (pBR322-gshIIbh)

By isolating recombinant plasmid pBR322-gsh IIb DNA (disclosed in Japanese Patent Application Disclosure SHO 60[1985)-180,592) in purified form by the method described in (1) of Item [1] from Escherichia coli RC912 (FERM BP-336) retaining a recombinant plasmid pBR322-gshIIb DNA there was

obtained 1 mg of a recombinant plasmid pBR322-gshIIb DNA.

By causing reaction of 2 μg of this recombinant plasmid pBR322-gshIIb DNA with 1 unit ot BamHI and 1 unit of HindIII (produced by Boehringer Mannheim-Yamanouchi K.K.) in 20 μl of a solution containing 50 mM Tris-HCl (pH 7.5)/10 mM $MgCl_2$/1 mM dithiothreitol/100 mM NaCl at 37°C for 1 hour, then subjecting the reaction solution to the conventional treatment of agarose gel electrophoresis thereby separating a DNA segment, cutting out the gel containing the DNA segment, placing the gel in dialytic tube, and subjecting the gel to the treatment of electrophoresis thereby removing the DNA segment out of the gel, there was obtained 0.6 μg of the DNA segment gshIIbb containing a GSH-II gene.

Then, 0.8 μg of various recombinant plasmid DNA's containing the recombinant plasmid pBR322-gshIIbh DNA were obtained by causing reaction of 0.5 μg of the plasmid pBR322 DNA with 1 unit of BamHI and 1 unit of HindIII in 20 μl of a solution containing 50 mM Tris-HCl (pH 7.5)/10 mM $MgCl_2$/1 mM dithiothreitol/100 mM NaCl at 37°C for 1 hour, adding to the resultant reaction solution 0.5 μg of the DNA segment gshIIbh obtained as described above and 1 unit of the T4 DNA ligase, and heating the resultant mixture at 16°C for 12 hours for the purpose of reaction.

Then, a transformed strain, i.e. E.coli 1100 (pBR322-gshIIbh), attaining no growth in a T-Y culture medium containing Tc in a concentration of 20 μg/ml was obtained by causing the E.coli 1100 treated in advance with calcium chloride to be transformed by the method described in (1) of Item [1] using the recombinant plasmid pBR322-gshIIbh DNA obtained as described above.

(2) Preparation of E.coli 1100 (pBR322-gshIIe)

By isolating a recombinant plasmid pBR322-gshIIbh DNA in purified form by the method described in (1) of Item [1] from the E.coli 1100 (pBR322-gshIIbh), there was obtained 1 mg of DNA.

By causing reaction of 1 μg of the pBR322-gshIIbh DNA with 2 units of BamHI in 20 μl of a solution containing 50 mM Tris-HCl (pH 7.5)/10 mM of $MgCl_2$/1 mM dithioerythritol/100 mM NaCl at 37°C for 1 hour, subjecting the resultant reaction solution to the conventional treatments of phenol extraction and ethanol precipitation thereby producing a precipitate, dissolving the precipitate in 20 μl of water, treating the resultant solution by the method described in "Molecular Cloning," pages 113 and 114, Cold Spring Harbor Laboratory (1982) using Klenow fragment E.coli DNA polymerase I (produced by Takara Shuzo K.K.), further adding thereto 0.1 μg of EcoRI linker, and treating the resultant mixture with 1 unit of the T4 DNA ligase as described above, there were obtained 0.9 μg of various recombinant plasmid DNA's containing the recombinant plasmid pBR322-gsh DNA.

A transformed strain, i.e. E.coli 1100 (pBR322-gshIIe), attaining growth on a T-Y culture medium containing Ap in a concentration of 20 μg/ml was obtained by causing the E.coli 1100 treated in advance with calcium chloride to be transformed by the method described in (1) of Item [1] using the recombinant plasmid pBR322-gshIIe DNA obtained as described above.

(3) Preparation of E.coli 1100 (pGSB403)

From the E.coli 1100 (pBR322-gshIIe), the recombinant plasmid pBR322-gshIIe DNA was isolated in purified form by the method described in (1) of Item [1], to obtain 1 mg of DNA.

By causing reaction of 2 μg of this pBR322-gshIIe with 2 units of DraII (produced by Boehringer Mannheim-Yamanouchi K.K.) and 2 units of EcoRI in 20 μl of a solution containing 33 mM Tris-acetic acid (pH 7.9)/10 mM magnesium acetate/0.5 mM dithiothreitol/66 mM potassium acetate at 37°C for 1 hour, then subjecting the resultant reaction solution to the conventional treatment of agarose gel electrophoresis thereby separating a DNA segment, cutting out the gel containing the DNA segment, placing the gel in a dialytic tube, subjecting it to the treatment of electrophoresis thereby removing the DNA segment out of the gel, there was obtained 0.6 μg of the DNA segment gshIIde containing a GSH-II gene.

Then, 0.8 μg of various recombinant plasmid DNA's containing a recombinant plasmid pGSB403 DNA were obtained by causing reaction of 0.5 μg of plasmid pUC19 DNA with 1 unit of SmaI (produced by Boehringer Mannheim-Yamanouchi K.K.) and 1 unit of EcoRI in 20 μl of a solution containing 33 mM Tris-hydrochloric acid (pH 7.9)/10 mM magnesium acetate/0.5 mM dithiothreitol/66 mM potassium acetate at 37°C for 1 hour, adding to the resultant reaction solution 0.5 μg of the DNA segment gshIIde obtained as described above and 1 unit of the T4 DNA ligase, and heating the resultant mixture at 16°C for 12 hours for the purpose of reaction.

Then, a transformed strain, i.e. E.coli JM101 (pGSB403), attaining growth on a T-Y culture medium containing Ap in a concentration of 20 μg/ml and forming a white colony on a T-Y culture medium containing 5-bromo-4-chloro-3-indolyl-β-D-galactopyranoside and isopropyl-β-D-thiogalactopyranoside was

obtained by causing E.coli JM101 treated in advance with calcium chloride to be transformed by the method described in (1) of Item [1] using the recombinant plasmid pGSB403 DNA obtained as described above.

(4) Preparation of E.coli 1100 (pAK223)

From E.coli JM101 (pAK122) (FERM BP-1534) retaining a recombinant plasmid pAK122 DNA, the recombinant plasmid pAK122 DNA was isolated in purified form by the method described (1) of Item [1], to obtain 1 mg of the DNA.

By causing reaction of 5 µg of this recombinant plasmid pAK122 DNA with 5 units of PstI (produced by Boehringer Mannheim-Yamanouchi K.K.) and 5 units of EcoRI in 20 µl of a solution containing 50 mM Tris-HCl (pH 7.5)10 mM $MgCl_2$/1 mM dithioerythritol/100 mM NaCl at 37°C for 1 hour, then subjecting the resultant reaction solution to the conventional treatment of agarose gel electrophoresis thereby separating the DNA segment, cutting out the gel containing the DNA segment, placing the gel in a dialytic tube, and subjecting this gel to the treatment of electrophoresis thereby removing the DNA segment out of the gel, there was obtained 2 µg of the DNA segment ack-pe containing an acetate kinase (hereinafter referred to briefly as "ACK").

Then, 1 µg of various recombinant plasmid DNA's containing the recombinant plasmid pAK223 DNA were obtained by causing reaction of 0.5 µg of the plasmid pBR322 DNA with 1 unit of PstI and 1 unit of EcoRI in 20 µl of a solution containing 50 mM Tris-HCl (pH 7.5)/10 mM $MgCl_2$/1 mM dithioerythritol/100 mM NaCl at 37°C for 1 hour, adding to the resultant reaction solution 1 µg of the DNA segment ack-pe obtained as described above and 1 unit of the T4 DNA ligase, and heating the resultant mixture at 16°C for 12 hours for the purpose of reaction.

A transformed strain, i.e. E.coli 1100 (pAK223), attaining growth on a T-Y culture medium containing Tc in a concentration of 20 µg/ml and attaining no growth on a T-Y culture medium containing Ap in a concentration of 20 µg/ml was obtained by causing the aforementioned E.coli 1100 treated in advance with calcium chloride to be transformed by the method described in (1) of Item [1] using the recombinant plasmid pAK223 DNA.

(5) Preparation of E.coli 1100 (pAK323)

From the E.coli JM101 (pGSB403) obtained by the procedure described in (3) of Item [4] and the E.coli 1100 (pAK223) obtained by the procedure described in (4) of Item [4], the plasmid pGSB403 DNA and the plasmid pAK223 DNA were respectively isolated in purified form by the method described in (1) of Item [1], to obtain 1 mg each of the DNA's.

Then, 2 µg of a DNA segment gshIIp containing a GSH-II gene was obtained by causing reaction of 5 µg of the recombinant plasmid pGSB403 DNA with 5 units of PvuII (produced by Boehringer Mannheim-Yamanouchi K.K.) in 20 µl of a solution containing 10 mM Tris-HCl (pH 7.5)/10 mM $MgCl_2$/1 mM dithioerythritol/50 mM NaCl at 37°C for 1 hour, then subjecting the resultant reaction solution to the conventional treatment of agarose gel electrophoresis thereby separating the DNA segment, cutting out the gel containing the DNA segment, placing the gel in a dialytic tube, and subjecting it to the treatment of electrophoresis thereby removing the DNA segment out of the gel.

Then, 1 µg of various recombinant plasmid DNA's containing a recombinant plasmid pAK323 DNA were obtained by causing reaction of 1 µg of the plasmid pAK223 DNA with 1 unit of HpaI (produced by Boehringer Mannheim-Yamanouchi K.K.) in 20 µl of a solution containing 33 mM Tris-acetic acid (pH 7.9)-/10 mM magnesium acetate/0.5 mM dithiothreitol/66 mM potassium acetate at 37°C for 1 hour, adding to the resultant reaction solution 1 µg of the DNA segment gshIIp and 1 unit of the T4 DNA ligase, and heating the resultant mixture at 16°C for 12 hours for the purpose of reaction.

A transformed strain, i.e. E.coli 1100 (pAK323), attaining growth on a T-Y culture medium containing Tc in a concentration 20 µg/ml was obtained by causing the aforementioned E.coli 1100 treated in advance with calcium chloride to be transformed by the method described in (1) of Item [1] using the recombinant plasmid pAK323 DNA.

The plasmid DNA was isolated by the method described in (5) of Item [1] from the transformed strain obtained as described above and then analyzed by agarose gel electrophoresis. In the consequently separated fractions of recombinant plasmid DNA, the recombinant plasmid having an ACK gene and a GSH-II gene incorporated simultaneously therein was found to be larger than the recombinant plasmid DNA having only an ACK gene incorporated therein. In other words, it was detected as a DNA of a short distance of movement in agarose gel electrophoresis. Thus, there was obtained a recombinant plasmid pAK323 DNA

possessing sectioned parts of EcoRI one each on the opposite outer sides of the GSH-II gene developed by the ACK gene and the lactose promoter.

(6) Isolation of recombinant plasmid pAK323 DNA

From the E.coli 1100 (pAK323), 0.7 mg of the recombinant plasmid pAK323 DNA was obtained by the method described in (1) of Item [1].

[5] Breeding of bacteriophage $\lambda cI_{857}$1121SΔRzKm

(1) Breeding of bacteriophage $\lambda cI_{857}$1121

A bacteriophage $\lambda cI_{857}$1121 was bred by faithfully following the procedure described in a working example cited in the specification of Japanese Patent Application Disclosure SHO 58(1983)-212,781.

This bacteriophage $\lambda cI_{857}$1121 has been deposited at Fermentation Research Institute, Agency of Industrial Science and Technology as E.coli ($\lambda cI_{857}$1121), namely a lysogen obtained by lysogenizing E.coli 1100 by the conventional method using the phage.

(2) Breeding of bacteriophage $\lambda cI_{857}$1121S

Bacteriophage particles were obtained by extracting a DNA by the method in popular use from the bacteriophage $\lambda cI_{857}$1121 obtained in (1) of Item [4], sectioning 2.1 $\mu$g of this DNA with 10 units of EcoRI (produced by Boehringer Mannheim-Yamanouchi K.K.) thereby obtaining a DNA segment, adding to the DNA segment 0.4 $\mu$g of the DNA segment obtained in advance by sectioning $\lambda cI_{857}$Sam7 DNA (procured from Washington Corp, USA) with 1 unit of the aforementioned EcoRI, then adding 1 unit of the T4 DNA ligase to the resultant mixture, heating the mixture at 7°C for 48 hours to obtain a mixture of recombinant DNA's, and enclosing the DNA mixture with the coating protein of bacteriophage by the in vitro packaging method.

Then, a bacteriophage $\lambda cI_{857}$1121S which possessed a property of forming no plaque when the E.coli 1100 described in (1) of Item [1] was used as an indicator bacterium but forming a plaque when E.coli QD5003 (obtained from Kyushu University) was used as an indicator bacterium, allowed the occurrence of a sectioned part by EcoRI at one position only in the DNA part ranging from the downsteam area of the anaphasic promoter $P'_R$ part to the cohesive end, and lacked the ability to dissolve the host bacterium because the S gene participating in lysogenization bactyeriolysis was recombined into the varied DNA segment (originating in a $\lambda cI_{857}$Sam7 DNA) was separated from the plaque produced by sprinkling the bacteriophage particles obtained as described above with the E.coli QD5003 as an indicator bacterium (used similarly hereinafter) and culturing them at 37°C for 16 hours.

(3) Preparation of E.coli 1100 (pKRq6)

A DNA segment was obtained from the bacteriophage $\lambda cI_{857}$1121S obtained in (2) of Item [4] by extracting the DNA from the bacteriophage by the method in popular use, sectioning 20 $\mu$g of the separated DNA with 10 units each of EcoRI and ClaI (both produced by Boehringer Mannheim-Yamanouchi K.K.), and subjecting the sectioned DNA to the conventional treatment of agarose gel electrophoresis.

By cutting out the gel containing the DNA segment, placing this gel in a dialytic tube, and subjecting it to the treatment of electrophoresis thereby removing the DNA segment out of the gel, there was obtained 0.5 $\mu$g of a DNA segment Rz-ce containing R and $R_z$ genes participating in bacteriolysis.

Then, a recombinant plasmid pKRq6 DNA retaining on a plasmid pBR322 DNA genes R and $R_z$ participating in bacteriolysis was obtained by mixing 0.5 $\mu$g of the DNA segment Rz-ce and 0.5 $\mu$g of the plasmid pBR322 DNA with 1 unit each of EcoRI and ClaI, heating the resultant mixture at 37°C for 2 hours for the purpose of digestion, combining the digested mixture with 1 unit of the T4 DNA ligase, and heating the resultant mixture at 16°C for 16 hours for the purpose of reaction and connection.

Subsequently, a strain, i.e. E.coli 1100 (pKRq6), attaining growth on a T-Y culture medium was obtained by causing the E.coli 1100 treated in advance with calcium chloride to be transformed by the method described in (1) of Item [1] using the recombinant plasmid pKRq6 obtained as described above.

(4) Preparation of E.coli 1100 (pKRq9)

By isolating the recombinant plasmid pKRq6 DNA in purified form by the method described in (1) of Item [1] from the E.coli 1100 (pKRq6) obtained in (3) of Item[4], there was obtained 0.7 mg of the DNA.

A transformed strain attaining growth on a T-Y culture medium containing Ap in a concentration of 20 µg/ml was obtained by mixing 5 µg of the recombinant plasmid pKRq6 DNA with 5 units of BclI (produced by Boehringer Mannheim-Yamanouchi K.K.), treating the resultant mixture at 37°C for 1 hour, then subjecting the treated mixture by the conventional treatments of phenol extraction and ethanol precipitation thereby producing a precipitate, dissolving the precipitate in 20 µl of water, treating the resultant aqueous solution with Bal31 by the method described in "Molecular Cloning," pages 135 to 139, Cold Spring Harbor Laboratory (1982), subjecting the treated solution to the treatment with phenol/chloroform and the treatment for ethanol precipitation as conventionally practised, dissolving the resultant precipitate in 20 µl of water, adding 10 µg of BglII linker (synthesized with a DNA synthesizing machine produced by Beckman) to the treated precipitate, causing 1 unit of the T4 DNA ligase to act on the resultant mixture at 16°C for 16 hours, and causing the E.coli 1100 treated in advance with calcium chloride to be transformed by the method described in (1) of Item [1] using the reaction product.

The recombinant plasmid DNA was isolated by the method described in (5) of Item [1] from the transformed strain and 1 µg of the plasmid DNA and 1 unit of BglII were treated at 37°C for 1 hour and subjected to the treatment of agarose gel electrophoresis. In the agarose gel electrophoresis, the plasmid DNA fragments separated by the distance of movement were measured in size. Consequently, there was isolated a plasmid pKRq70 DNA deprived of about 200 bp of the $R_z$ gene participating in bacteriolysis.

From the transformed strain 1100 (pKRq70) arising from the plasmid pKRq70 DNA, 0.6 mg of the recombinant plasmid pKRq70 DNA was isolated in purified form by the method described in (1) of Item [1].

Then, 0.3 µg of the DNA segment cos-bb of about 1.7 kb was obtained by causing reaction of 2 µg of cosmid pHC79 DNA (produced by Boehringer Mannheim-Yamanouchi K.K.) with 5 units of BglII in 20 µl of a solution containing 10 mM Tris-HCl (pH 7.5)/10 mM $MgCl_2$/1 mM dithioerythritol/50 mM NaCl at 37°C for 1 hour and subsequently treating the resultant reaction solution by the method described in (4) of Item [1].

Then, 0.5 µg of various recombinant cosmid DNA's and plasmid DNA's containing the recombinant cosmid pKRq8 DNA were obtained by causing reaction of 0.5 µg of the plasmid pKRq7 DNA obtained as described above with 1 unit of BamHI and 1 unit of BglII in a solution containing 10 mM Tris-HC1 (pH 7.5)-/10 mM $MgCl_2$/1 mM dithioerythritol/50 mM NaCl at 37°C for 1 hour, subjecting the resultant reaction solution to the treatment with phenol and the treatment for ethanol precipitation as conventionally practised thereby producing a precipitate, dissolving the precipitate in 20 µl of water, adding to the solution 0.3 µg of the DNA segment cos-bb obtained as described above and 2 unit of the T4 DNA ligase, and heating the resultant mixture at 16° C for 12 hours for the purpose of reaction.

A transformed strain was obtained by causing the E.coli 1100 treated in advance with calcium chloride to be transformed by the method described in (1) of Item [1] using the aforementioned recombinant DNA.

The recombinant cosmid DNA isolated by the method described in (5) of Item [1] from the transformed strain mentioned above was analyzed by agarose gel electrophoresis. As the result, the recombinant cosmid incorporating the DNA segment cos-bb therein was separated as a larger cosmid DNA than the other plasmid DNA and cosmid DNA. In this manner, there was obtained an E.coli 1100 (pKRq8), a strain transformed by the cosmid pKRq8 DNA retaining the genes S and R participating in bacteriolysis and deprived of the gene Rz participating in bacteriolysis.

By mixing 0.5 µg of the recombinant cosmid pKRq8 DNA obtained as described above with 1 unit of BglII, heating the resultant mixture at 37°C for 1 hour for the purpose of digestion, subjecting the resultant digested solution to the treatment with phenol and the treatment for ethanol precipitation as generally practised thereby producing a precipitate, dissolving the precipitate in 20 µl of water, treating the resultant aqueous solution with the Klenow fragment of E.coli DNA polymerase I in accordance with the method described in (1) of Item [1], adding 1 µg of XbaI linker to the treated solution, and causing 1 unit of the T4 DNA ligase to react on the resultant mixture at 16°C for 16 hours, there were obtained 0.4 µg of various recombinant cosmids containing the recombinant cosmid pKRq9 DNA.

A transformed strain, i.e. E.coli 1100 (pKRq9), was obtained by causing the E.coli 1100 treated in advance with calcium chloride to be transformed by the method described in (1) of Item [1] using the recombinant cosmid pKRq9 DNA.

(5) Preparation of E.coli 1100 (pUCKm)

In a solution containing 50 mM Tris-HCl (pH 7.5)/10 mM $MgCl_2$/1 mM dithiothreitol/100 mM NaCl, 1 $\mu$g of plasmid vector pUC-4K DNA (produced by Farmacia) and 2 units of EcoRI were left reacting at 37°C for 1 hour. The reaction solution was subjected to the conventional method of phenol extraction and ethanol precipitation. Consequently, there was obtained 0.8 $\mu$g of the product of digestion of the plasmid vector pUC-4K DNA with EcoRI.

Then, 0.8 $\mu$g of a plasmid vector pUCKm DNA was obtained by treating 0.8 $\mu$g of the product of digestion of the plasmid vector pUC-4K DNA with EcoRI in accordance with the method described in (2) of Item [3] using Klenow fragment of E.coli DNA polymerase I, further adding 1 $\mu$g of the XbaI linker (produced by New England Biolabs) and 1 unit of the T4 DNA ligase to the treated digestion product, and allowing the reactants to react at 4°C for 20 hours.

An E.coli 1100 (pUCKm) was obtained by causing the E.coli 1100 treated in advance with calcium chloride to be transformed by the method described in (1) of Item [1] using the plasmid vector pUCKm DNA obtained as described above.

(6) Preparation of E.coli 1100 (pKRq10)

By obtaining 1 mg of a plasmid vector pUC-4K DNA in entirely the same manner as described in (1) of Item [1] from the E.coli 1100 (pUCKm) obtained in (5) of Item [5], causing reaction of 10 $\mu$g of the DNA with 20 units of XbaI in a solution containing 50 mM Tris-HCl/10 mM $MgCl_2$/1 mM dithioerythritol/100 mM NaCl at 37°C for 1 hour, subjecting the resultant reaction solution to the treatment of agarose gel electrophoresis, and treating the treated solution by following the procedure described in (5) of Item [1], there was obtained 3 $\mu$g of the DNA segment Kmxb of 1.4 Kb containing a Kanamycin-resistant gene.

Subsequently, a transformed strain, i.e. E.coli 1100 (pKRq10), attaining growth on a T-Y agar culture medium containing Km in a concentration of 20 $\mu$g/ml was obtained by obtaining 0.7 mg of a cosmid pKRq9 DNA in entirely the same manner as described in (1) of Item [1] from the E.coli 1100 (pKRq9) obtained in (4) of Item [5], allowing 1 $\mu$g of this DNA to react with 1 unit of XbaI in a solution containing 50 mM Tris-HCl/10 mM $MgCl_2$/1 mM dithioerythritol/100 mM NaCl at 37°C for 1 hour, then subjecting the resultant reaction solution to the treatment with phenol and the treatment for ethanol precipitation as generally practised thereby producing a precipitate, dissolving the precipitate in 20 $\mu$l of water, adding 0.8 $\mu$g of the DNA segment Kmxb obtained above to the resultant aqueous solution, allowing 1 unit of the T4 DNA ligase to react on the resultant mixture at 16°C for 16 hours, and causing the E.coli 1100 treated in advance with calcium chloride to be transformed in accordance with the method described in (1) of Item [1] using the resultant reaction product.

(7) Breeding of bacteriophage $\lambda cI_{857}$1121S$\Delta$RzKm

By isolating a recombinant cosmid pKRq10 DNA in purified form in accordance with the method described in (1) of Item [1] from the E.coli 1100 (pKRq10) obtained in (6) of Item [5], there was obtained 0.7 mg of the DNA.

A mixture of 10 $\mu$g of the recombinant cosmid pKRq10 DNA and 10 $\mu$g of the bacteriophage $\lambda cI_{857}$1121S DNA obtained in (2) of Item [5] with 50 units of EcoRI was heated at 37°C for 1 hour and then subjected to the treatment with phenol and the treatment for ethanol precipitation as generally practised. The precipitate consequently formed was dissolved in 10 $\mu$l of water. The aqueous solution and 2 units of the T4 DNA ligase added thereto were left reacting at 4°C for 48 hours.

Bacteriophage particles were prepared by the in vitro packaging method described in Item [3] using 10 $\mu$g of the DNA. In 50 $\mu$l of the solution of phage particles, E.coli 1100 ($10^9$/ml, 0.5 $\mu$l) was incubated at 30°C for 2 hours. The incubated microorganism was sprinkled on T-Y agar culture medium containing Km in a concentration of 20 $\mu$g/ml and cultured at 32° C for 24 hours, to obtain a lysogen 1100 ($\lambda cI_{857}$1121S$\Delta$RzKm) as a Km-tolerant strain.

As described above, there was obtained by separation the bacteriophage $\lambda cI_{857}$1121S$\Delta$RzKm which allowed occurrence of a part sectioned by EcoRI at one position only in the DNA part ranging from the downstream area of the anaphasic promoter $P'_R$ part to the cohesive end, suffered loss of the ability to dissolve the host bacterium because the gene participating in bacteriolysis was recombined into the DNA segment (originating in $\lambda cI_{857}$Sam7 DNA) owing to variation of the gene and the Rz gene participating in bacteriolysis was lost, and yet retained a Kanamycin-resistant gene. [6] Preparation of recombinant bacteriophage EN1121S$\Delta$RzKm-ACKGSH II (lacP) having ACK and GSH-II genes inserted under control of

DNA arrangement necessary for translation in endonuclease sectioned part present in DNA part ranging from downstream area of anaphasic promoter of bacteriophage $\lambda cI_{857}$1121SΔRzKm to cohesive end.

From the bacteriophage obtained in (7) of Item [5], the DNA was extracted by the conventional method. A mixture of 10 µl of this $\lambda cI_{857}$1121SΔRzKm DNA with 3 µg of the recombinant plasmid pAK323 DNA obtained in (6) of Item [4] was placed in 50 µl of a solution of the composition of 50 mM Tris-HCl (pH 7.4)-/100 mM $MgCl_2$/20 mM $MgSO_4$, reacted upon by 50 units of EcoRI at 37 °C for 2 hours, and then subjected to the treatments of phenol extraction and ethanol precipitation as generally practised to produce a precipitate. The precipitate was placed in 8 µl of a solution of the composition of 50 mM Tris-HCl (pH 7.4)-/10 mM dithiothreitol/10 mM $MgCl_2$/0.1 mM ATP and left standing at 4 °C for 18 hours for the purpose of inducing a connecting reaction.

Bacteriophage particles were prepared by enclosing 10 µg of the DNA obtained as described above with a coating protein of bacteriophage in accordance with the in vitro packaging method. A mixture of 50 µl of the phage particles with E.coli 1100 ($10^9$/ml, 0.5 µl) was left incubating at 30 °C for 2 hours, sprinkled on a T-Y agar culture medium containing Kanamycin (Km) in a concentration of 10 µg/ml, and cultured at 32 °C for 24 hours.

In the bacterial strains cultured and grown as described above, the lysogen produced by the recombinant bacteriophage DNA retaining an ACK gene and a GSH-II gene on the bacteriophage $\lambda cI_{857}$1121SΔRzKm DNA was screened by the following method, there was obtained a lysogen possessing an ACK gene and a GSH-II gene on several strains ot bacteriophage DNA.

The lysogen obtained as described above, namely the bacterium tolerant to Kanamycin and sensitive to temperature was inoculated to a T-Y medium and shaken cultured at 32 °C for 16 hours. A 0.5-ml inoculum of the resultant culture broth was inoculated to a 10-ml T-Y culture medium in a conical flask having an inner volume of 150 ml and, after the number of Klett units reached about 100, heated to 43 °C and shaken at this temperature for 25 minutes, then cooled to 32 °C and shaken at this temperature for about 3 hours.

A 1-ml portion of the consequently obtained culture broth was crushed by ultrasonic wave to obtain a cell extract. This cell extract was tested for enzymatic activity of GSH-II in accordance with the method described in "Journal of General Microbiology," vol. 128, pages 1047 to 1052 (1982) and further tested for enzymatic activity of ACK by the method described in "Journal of Bacteriology," vol. 144, pages 672 to 682 (1980) to select a strain of high enzymatic activities. This highly active lysogen was inoculated to a 10-ml T-Y culture medium, shaken cultured at 32 °C until the number of Klett units rose to about 100, then heated at 43 °C for 20 minutes, and further shaken cultured at 37 °C for 3 hours. From the resultant cultured broth, DNA was extracted by using the same volume of a mixed solvent of phenol/chloroform as the culture broth. The extracted DNA was subjected to precipitation with ethanol, to obtain the DNA.

The DNA thus obtained was dissolved in 1 ml of a solution of the composition of Tris-HCl (pH 7.5)/1 mM EDTA. In 30 µl of a solution of the composition of 10 mM Tris-HCl (pH 7.4)/100 mM NaCl/10 mM $MgSO_4$/1 mM dithiothreitol, 4 µl of the resultant solution was placed and reacted upon by 50 units of EcoRI and 20 µg of RNaseA (produced by Sigma K.K.) at 37 °C for 1 hour for the purpose of digestion. The product of this digestion was subjected to the treatment of agarose gel electrophoresis for the purpose of analyzing size of DNA segment.

As the result, the DNA segment originating in the recombinant plasmid pAK323 DNA of about 4 Kbp was detected in all of the lysogens of high activity of ACK and GSH-II.

By isolating the E.coli 1100 (EN1121SΔRzKm-ACKGSH II (lacP)), the recombinant bacteriophage EN1121SΔRzKm-ACKGSH II (lacP) DNA was produced.

[7] Production of bacteriophage 501GI (lacP) 2ACKIG II (lacP)1 retaining GSH-I, ACK, and GSH-II genes on one and the bacteriophage DNA

(1) Preparation of E.coli 1100 [501GI (lacP) 2ACKIG II (lacP)1]

From the E.coli 1100 (EN501SGI (lacP)2) obtained as described in Item [3], the bacteriophage EN501SGI (lacP)2 DNA was extracted in accordance with the method described in (3) of Item [2]. Similarly, the bacteriophage EN1121SΔRzKm-ACKGSH II (lacP) DNA was extracted from the E.coli 1100 (EN1121SΔRzKm-ACKGSH II (lacP) obtained as described in Item [6].

Then, a mixture of 10 µg of the bacteriophage EN501SGI (lacP)2 DNA and 10 µg of the bacteriophage EN1121SΔRzKm-ACKGSH II (lacP) DNA was placed in 50 µl of a solution of the composition of 10 mM Tris-HCl (pH 7.5)/10 mM $MgCl_2$/1 mM dithioerythritol/50 mM NaCl, then reacted upon by 50 units of NheI (produced by Boehringer Mannheim-Yamanouchi K.K.) at 37 °C for 2 hours, and subjected to the conventional treatments of phenol extraction and ethanol precipitation to produce a precipitate. The precipitate was

placed in 8 µl of a solution of the composition of 50 mM Tris-HCl (pH 7.4)/10 mM $MgCl_2$/10 mM dithiothreitol/0.1 mM ATP and further reacted upon by 2 units of the T4 DNA ligase at 4 ° C for 18 hours for the purpose of a connecting reaction.

A lysogen was obtained by enclosing 10 µg of the DNA consequently obtained with the coating protein of λ bacteriophage by the in vitro packaging method thereby preparing bacteriophage particles, allowing 50 µl of the phage particles and E.coli 1100 ($10^9$/ml, 0.5 µl) added thereto to stand at 30 ° C for 2 hours for the purpose of incubation, sprinkling the incubated microorganism on a T-Y agar culture medium containing Km in a concentration of 10 µg/ml, and culturing the microorganism at 32 ° C for 24 hours.

The lysogen containing the recombinant DNA was selected as a strain gaining growth in a culture medium containing Km in a concentration of 10 µg/ml, attaining no growth in a culture medium containing Tc in a concentration of 1.5 µg/ml, and lacking an ability to form a plaque. The method for detecting the recombinant DNA in the selected strain containing the recombinant DNA is as shown below.

The selected lysogen containing the recombinant DNA was inoculated to 1 ml of a T-Y culture medium, cultured until the number of Klett units reached about 100, then heated to 43 ° C and shaken cultured at this temperature for 25 minutes, and cooled to 32 ° C and shaken cultured at this temperature for about 3 hours.

The DNA was extracted from the consequently obtained culture broth by placing the culture broth in 50 µl of chloroform, continuing the shaken culture of the microorganism in the resultant mixture at 32 ° C for 20 minutes (which operation brought about bacteriolysis), and subjecting the shaken mixture to the conventional treatments of phenol extraction and ethanol precipitation.

The DNA obtained as described above was treated with EcoRI and KpnI in the manner described above and analyzed by agarose electrophoresis for DNA segments to determine size of DNA segment. Consequently, the recombinant DNA retained by the selected lysogen was found to retain the DNA segments originating in the pGS1102 containing two GSH-I genes and pAK323 containing an ACK gene and a GSH-II gene.

The E.coli 1100 (501GI (lacP) 2ACKIG II (lacP)1) was separated as described above.

The E.coli 1100 (501GI (lacp) 2ACKIG II (lacP) 1) has been deposited at Fermentation Research Institute, Agency of Industrial Science and Technology as FERM P-10602 (BP-2733).

(2) Isolation of recombinant bacteriophage 501GI (lacP) 2ACKIG II (lacP)1 DNA

A recombinant bacteriophage 501GI (lacp) 2ACKIG II (lacP)1 DNA was obtained in purified form in an amount of 500 µg by inoculating the lysogen, i.e. E.coli 1100 (501GI (lacP) 2ACKIG II (lacP)1, obtained as described above to 3 ml of a T-Y culture medium, shaken culturing at 32 ° C for 16 hours, placing 500 µl of the resultant culture broth to 30 ml of a T-Y culture medium in a conical flask having an inner volume of 500 ml, shaken culturing the microorganism at 32 ° C until the number of Klett units reached about 100, heating at 42 ° C for 15 minutes thereby effecting thermal induction, subsequently shaken culturing at 37 ° C for 2 hours, combining the resultant culture broth with 500 µl of chloroform (which operation brought about bacteriolysis) thereby producing a lysogen, placing 2 ml of the lysogen in 400 µl of a solution containing 0.25 M EDTA/0.5 M Tris-HC (pH 9.0)/2.5% sodium dodecyl-sulfate, heating at 70 ° C for 30 minutes, subsequently adding 500 µl of an aqueous 8 M potassium acetate solution, allowing the resultant mixture to stand on ice for 15 minutes, subjecting the cooled mixture to centrifugal separation at 12,000 g for 20 minutes, separating the supernatant, diluting this supernatant with twice as large in volume of ethanol, centrifugally separated at 27,000 g for 30 minutes, and drying the resultant precipitate by the method in popular use. The limiting enzyme cleavage map of the recombinant bacteriophage DNA is shown in Fig. 1. In the diagram, K, E, N, and X respectively stand for the limiting enzymes KpnI, EcoRI, NheI, and XbaI.

(3) GSH-I, GSH-II, and ACK activities of cell bodies obtained by culturing lysogen due to recombinant bacteriophage EN1121SΔRzKmACKGSH II (lacP) DNA, bacteriophage EN501SI (lacP)2 DNA, and bacteriophage 501GI (lacP)2ACKIG II (lacP)1 DNA.

The GSH-I, GSH-II, and ACK activities possessed by the lysogens, E.coli 1100 (EN1121SΔRzKm-ACKGSH II (lacP)), E.coli 1100 (EN501SGI (lacP)2), and E.coli 1100 (501GI (lacP)2ACKIG II (lacP)1), obtained as described above are shown in Table 1.

The enzyme activities were each determined of a cell extract prepared from cell bodies cultured in a T-Y culture medium in accordance with the method described in Item [6].

Further, the enzyme activity was determined by the method described in "Journal of General Microbiology," vol. 128, pages 1047 to 1052 (1982) and "Journal of Bacteriology," vol. 144, pages 672 to 682 (1980).

Table 1

| Kind of strain | Enzyme activity (μ•mol/h./mg of protein) | | |
|---|---|---|---|
| | GSH-I | GSH-II | ACK |
| E.coli 1100 (EN1121SΔRzKmACKGSH II(lacP) (Control) | 0.4 | 11.0 | 123.1 |
| E.coli 1100 (EN501SGI (lacP)2) (Control) | 22.8 | 0.4 | 2.8 |
| E.coli 1100 (501GI(lacP)2ACKIG II(lacP)1) (This invention) | 21.6 | 70.3 | 150.6 |

[8] Glutathione forming activity

(1) Production of bacteriophage 501GI (lacP)2G II (lacP)1 retaining GSH-I and GSH-II genes on one and same bacteriophage DNA

A strain, E.coli JM101 (pGSB420), transformed with the plasmid pGSB420 possessing EcoRI sectioned parts one each on the opposite outer sides of a GSH-II gene was obtained by treating 1 μg of the plasmid pGSB402 DNA obtained as described in (3) of Item [4] with 1 unit of PvuII at 37°C for 1 hour, then treating 1 μg of the plasmid vector pUC18 DNA (produced by Takara Shuzo Co., Ltd.) with 1 unit of SmaI at 37°C for 1 hour, subjecting the treated DNA's to a connecting reaction by the conventional method using the T4 DNA ligase, and transforming the connected DNA's by the method described in (1) of Item [1]. By isolating the plasmid pGSB420 DNA in purified form from the E.coli JM101 (pGSB420) in accordance with the method described in (1) of Item [1], there was obtained 1 mg of the DNA.

Then, the recombinant bacteriophage EN1121SΔRzKmGSH II (lacP) DNA possessing a GSH-II gene on the bacteriophage DNA was prepared from the bacteriophage $\lambda cI_{857}$1121SΔRzKm DNA and the plasmid pGSB420 DNA by following the procedure described in Item [6].

Subsequently, a bacteriophage 501GI (lacP) 2G II (lacP)1 DNA possessing a GSH-I gene and a GSH-II gene on one and the same phage DNA and a lysogen, E.coli 1100 (501GI (lacP) 2G II (lacP)1) were obtained by recombining the bacteriophage EN1121SΔRzKmGSH II (lacP) DNA and the bacteriophage EN501GI (lacP)2 DNA by following the procedure described in Item [7].

(2) GSH-I, ACK, and GSH-II activities and glutathione forming activity of cell bodies obtained by culturing lysogen due to recombinant bacteriophage 501GI (lacP) 2ACKIG II (lacP)1 DNA and bacteriophage 501GI-(lacP)2G II (lacP)1 DNA.

The GSH-I, GSH-II, and ACK activities possessed by the lysogens, i.e. E.coli 1100 (501GI (lacP)2ACKIG II (lacP)1) and E.coli 1100 (501GI (lacP)2G II (lacP)1), obtained as described above were measured in the same manner as described in (3) of Item [7]. The results are shown in Table 2.

Table 2

| Kind of strain | Enzyme activity (μ•mol/h./mg of protein) | | |
|---|---|---|---|
| | GSH-I | GSH-II | ACK |
| E.coli 1100 (501GI(lacP)2G II(lacP)1) | 18.7 | 36.5 | 2.8 |
| E.coli 1100 (501GI(lacP)2ACKIG II(lacP)1) | 21.6 | 70.3 | 150.6 |

The lysogens, i.e. E.coli 1100 (501GI (lacP)2ACKIG II (lacP)1) and E.coli 1100 (501GI (lacP)2G II (lacP)-1), were cultured in accordance with the method described in Item [6] using a T-Y culture medium and E.coli 1100 was shaken cultured in a T-Y culture medium at 37°C for 20 hours. The cell bodies thus obtained were collected and washed once with 0.85% (W/V) physiological saline solution. 100-mg of such cell bodies was suspended in 2 ml of a 5 mM Tris-HCl (pH 7.0) buffer solution containing 0.5 mM L-cysteine and subjected to the conventional treatment of supersonic pulverization. A crude enzyme extract containing 1 mg of protein and a reaction solution containing 80 mM L-glutamic acid, 20 mM L-cysteine, 20 mM glycine, 20 mM magnesium chloride, 0.5 or 20 mM ATP, 20 or 39.5 mM acetyl phosphoric acid, and

25 mM potassium phosphate (pH 7.2) buffer solution added thereto in such an amount as to total 1 ml were shaken cultured at 37°C for 1 hour. The glutathione forming activity was calculated from the amount of glutathione formed in the final reaction solution. The results are shown in Table 3.

Table 3

| Kind of strain | Glutathione forming activity (μ·mol/mg of protein/h.) | |
|---|---|---|
| | ATP 20mM Acetyl phosphoric acid 20mM | ATP 0.50mM Acetyl phosphoric acid 39.5mM |
| E.coli 1100 (Control) | 0.2 max. | 0.2 max. |
| E.coli 1100 (501GT(l--cP) 2GII (lacP)1) (Control) | 24.1 | 16.7 |
| E.coli 1100 (501GI(lacP)2ACKIG II(lacP)1) (This invention) | 20.8 | 22.8 |

[9] Preparation of recombinant plasmid pCK9-3 DNA

(1) Preparation of E.coli JM101 (pCK 0)

A solution of a c-DNA liberary (produced by Clontech and obtained from Toyobo Co., Ltd.) originating in rat brain, with E.coli Y1090 (obtained from Clontech) as an indicator bacterium, was sprinkled on a Trypton agar culture medium [containing 1% Trypton® (produced by Difco), 0.25% NaCl, and 1.2% agar, sterilized with an autoclave, and dispensed in fractions of 30 ml to petri dishes 9 cm in diameter], subjected to stationary culture at 37°C for 14 hours, to obtain about 50,000 bacteriolytic strains.

The nucleotide sequence of the rat brain creatine kinase (hereinafter referred to briefly as "CK") gene is described in the report by P.A. Benfield in "Gene," vol. 39, pages 263 to 267 (1985). The 17-base oligonucleotide of 5′-TGCTGACCCCCGAGCTG-3′, one part of the nucleotide sequence of the CK gene, was synthesized with a DNA synthesizer machine (produced by Beckman). The 5′ end of this 20ng oligonucleotide was labeled with [$\gamma$ -$^{32}$P] ATP [produced by Amersham] in accordance with the method described in "Molecular Cloning," pages 122 to 126, Cold Spring Harbor Laboratory (1982).

By using the oligonucleotide labeled with the $^{32}$P as a probe and screening the aforementioned c-DNA library by the plaque hybridization method ["Molecular Cloning," pages 312 to 328, Cold Spring Harbor Laboratory (1982)], a plaque possessing a CK gene was obtained. A bacteriophage DNA containing a CK gene was produced in purified form in accordance with the method described in "Molecular Cloning," pages 371 and 372, Cold Spring Harbor Laboratory (1982) using this plaque. This recombinant bacteriophage DNA was named λgtll-ck.

A DNA segment $CK_{EC}$ containing a CK gene was obtained in an amount of 0.5 μg by causing reaction of 5 μg of the recombinant bacteriophage λgtll-CK DNA with 10 units of EcoRI (produced by Boehringer Mannheim-Yamanouchi K.K.) in 30 μl of a solution containing 50 mM Tris-HCl (pH 7.5)/10 mM $MgCl_2$/1 mM dithioerythritol/100 mM NaCl at 37°C for 1 hour, subjecting the resultant reaction solution to the conventional treatment of agarose gel electrophoresis thereby separating a DNA segment of about 1.3 Kb, cutting out the gel containing the DNA segment, placing the gel in a a dialytic tube, and subjecting it to the treatment of electrophoresis thereby removing the DNA segment out of the gel.

A DNA segment $CK_{ECF}$ possessing smooth ends was obtained in an amount of 0.4 $\mu$g by dissolving 0.5 $\mu$g of the $CK_{EC}$ in 20 $\mu$l of water and treating the resultant aqueous solution in accordance with the method described in "Molecular Cloning," pages 113 and 114, Cold Spring Harbor Laboratory (1982) using a Klenow fragment of E.coli DNA polymerase I (produced by Takara Shuzo Co., Ltd.).

Then, a DNA segment $pUC119_{XF}$ possessing flat ends was obtained in an amount of 0.4 $\mu$g by causing reaction of 0.5 $\mu$g of the plasmid pUC119 DNA (produced by Takara Shuzo Co., Ltd.) with 5 units of XbaI (produced by Boehringer Mannheim-Yamanouchi K.K.) in 20 $\mu$l of solution containing 50 mM Tris-HCl (pH 7.5)/10 mM $MgCl_2$/1 mM dithioerythritol/100 mM NaCl at 37°C for 1 hour, then subjecting the resultant reaction solution to the treatment with phenol and the treatment for ethanol precipitation as generally practised thereby producing a precipitate, treating the precipitate, similarly to the aforementioned $CK_{EC}$ DNA segment, with Klenow fragment of E.coli DNA polymerase I (produced by Takara Shuzo Co., Ltd.).

A recombinant plasmid pCKO DNA capable of expressing a fused protein of $\beta$-galactosidase and CK under control of the lac promoter on the plasmid 119 DNA was obtained by dissolving 0.4 $\mu$g of the $CK_{ECF}$ DNA segment and 0.4 $\mu$g of $pUC119_{XF}$ DNA segment in 10 $\mu$l of water and allowing the resultant aqueous solution and the T4 DNA ligase (produced by Boehringer Mannheim-Yamanouchi K.K.) added thereto to react at 4°C for 24 hours thereby effecting a connecting reaction in accordance with the method described in "Molecular Cloning," pages 396 and 397, Cold Spring Harbor Laboratory (1982).

Then, a transformed strain, i.e. E.coli JM101 (pCK 0), attaining growth on a T-Y culture medium containing Ap in a concentration of 20 $\mu$g/ml and forming a white colony on a T-Y culture medium containing 5′-bromo-4-chloro-3-indolyl-$\beta$-D-galactopyranoside and isopropyl-$\beta$-thiogalactopyranoside was obtained by causing the E.coli JM101 treated in advance with calcium chloride to be transformed by the method described in (1) of Item [1] using the recombinant plasmid pCK 0 DNA obtained as described above.

(2) Preparation of plasmid vector pCK 8 DNA

A plasmid pCK 8 DNA possessing sectioned parts of KpnI, BglII, and SmaI on the plasmid DNA was obtained by mixing 1 $\mu$g of a plasmid pBR322 DNA (produced by Takara Shuzo Co., Ltd.) with 2 units of EcoRI (produced by Boehringer Mannheim-Yamanouchi K.K.) and 3 units of SalI (produced by Boehringer Mannheim-Yamanouchi K.K.), heating the resultant mixture at 37°C for 1 hour for the purpose of digestion, subjecting the digested mixture to the treatment with phenol and the treatment for ethanol precipitation as generally practised thereby giving rise to a precipitate, dissolving the precipitate in 17 $\mu$l of water, mixing the resultant aqueous solution with 0.3 $\mu$g each of a 29-base oligonucleotide of 5′-AATTGGTACCAGATCTCCCGGGGGTACCG-3′ and a 29-base oligonucleotide of 5′-TCGACGGTACCCCCGGGAGATCTGGTACC synthesized with a DNA synthesizer machine (produced by Beckman), and causing 1 unit of the T4 DNA ligase to react upon the resultant mixturte at 20°C for 16 hours.

A transformed strain, i.e. E.coli JM101 (pCK 8), attaining growth on a T-Y culture medium containing Ap in a concentration of 20 $\mu$g/ml and attaining no growth on a T-Y culture medium containing tetracycline (Tc) in a concentration of 20 $\mu$g/ml was obtained by causing the E.coli JM101 (produced by Takara Shuzo Co., Ltd.) treated in advance with calcium chloride to be transformed by the method described in (1) of Item [1] using the aforementioned plasmid DNA.

By isolating the plasmid pCK 8 DNA in purified form by the method described in (1) of Item [1] from the consequently obtained E.coli JM101 (pCK 8), there was obtained 1 mg of pCK 8 DNA.

(3) Preparation of E.coli JM101 (pCK 9)

By isolating the recombinant plasmid pCK 0 DNA in purified form by the method described in (1) of Item [1] from the E.coli JM101 (pCK 0) obtained in (1) of Item [9], there was obtained 1.1 mg of the pCK 0 DNA.

A 1.5 Kb DNA segment lac-CK containing a lactose promoter and a CK gene was obtained in an amount of 1.2 $\mu$g by causing reaction of 7 $\mu$g of the recombinant plasmid pCK 0 obtained as described above with 10 units of PvuII (produced by Boehringer Mannheim-Yamanouchi K.K.) in 20 $\mu$l of a solution containing 10 mM Tris-HCl (pH 7.5)/10 mM $MgCl_2$/1 mM dithioerythritol/50 mM NaCl at 37°C for 2 hours, then subjecting the resultant reaction solution to the conventional treatment of agarose gel electrophoresis thereby separating about 1.5 Kb DNA segment, placing the DNA segment in a dialytic tube, and subjecting to the treatment of electrophoresis thereby removing the DNA segment out of the gel.

Then, a recombinant plasmid pCK 9 DNA was obtained by causing reaction of 1 $\mu$g of the plasmid pCK 8 DNA obtained as described in (2) of Item [9] with 2 units of SmaI (produced by Boehringer Mannheim-Yamanouchi K.K.) in 20 µl of a solution containing 33 mM Tris-acetate (pH 7.9)/10 mM magnesium acetate/66 mM potassium acetate/0.5 mM dithiothreitol at 30 °C for 2 hours, then subjecting the resultant reaction solution to the treatment with phenol and the treatment for ethanol precipitation as generally practised thereby giving rise to a precipitate, dissolving the precipitate in 20 µl of water, mixing the resultant aqueous solution with 1.0 µg of the lac-CK DNA segment obtained as described above and 1 unit of the T4 DNA ligase, and allowing the resultant mixture to stand at 4 °C for 24 hours for the purpose of reaction.

This pCK 9 DNA was a recombinant plasmid possessing sectioned parts of KpnI BglII in the lactose promoter and in the upstream part of the lactose promoter of the CK gene and sectioned parts of BamHI and KpnI in the downstream part ot the CK gene.

Then, a transformed strain, i.e. E.coli JM101 (pCK 9), attaining growth on a T-Y culture medium containing Ap in a concentration of 20 µg/ml was obtained by causing the E.coli JM101 treated in advance with calcium chloride to be transformed by the method described in (1) of Item [1] using the plasmid DNA mentioned above.

(4) Preparation of E.coli JM101 (pCK 9-3)

By isolating the recombinant plasmid pCK 9 DNA in purified form by the method described in (1) of Item [1] from the E.coli JM101 (pCK 9), there was obtained 1.2 mg of the pCK 9 DNA.

A DNA segment CK $(lac\text{-}P)_{BB}$ containing lactose promoter and a CK gene was obtained in an amount of 2 µg by causing reaction of 6 µg of the recombinant plasmid pCK 9 DNA obtained as described above with 6 units of BamHI and 6 units of BglII (both made by Boehringer Mannheim-Yamanouchi K.K.) in 60 µl of a solution containing 10 mM Tris-HCl (pH 8.0)/5 mM $MgCl_2$/100 mM NaCl at 37 °C for 2 hours, subjecting the resultant reaction solution to the conventional treatment of agarose gel electrophoresis thereby separating a DNA segment, cutting out the gel containing the about 1.5 Kb DNA segment, placing the gel in a dialytic tube, subjecting it to the treatment of electrophoresis thereby removing the DNA segment out of the gel.

Then, various recombinant plasmid DNA's containing the recombinant plasmid pCK9-3 DNA were obtained in an amount of 2 µg by causing reaction of 1 µg of the recombinant plasmid pCK9-3 DNA with 1 unit of BamHI (produced by Boehringer Mannheim-Yamanouchi K.K.) in 20 µl of a solution of the same composition as described above at 37 °C for 1 hour, then subjecting the resultant reaction solution to the treatment with phenol and the treatment for ethanol precipitation as generally practised thereby giving rise to a precipitate, dissolving the precipitate in 20 µl of water, and allowing 2 µg of the aforementioned DNA segment CK $(lac\text{-}P)_{BB}$ and 1 unit of the T4 DNA ligase to react upon the resultant aqueous solution at 16 °C for 12 hours.

Subsequently, a transformed strain, i.e. E.coli JM101 (pCK 9-3), attaining growth on a T-Y culture medium containing Ap in a concentration of 20 µg/ml was separated by causing the E.coli JM101 treated in advance with calcium chloride to be transformed by the method described in (1) of Item [1] using the plasmid DNA mentioned above. By the agarose gel electrophoresis described in (5) of Item [1], the E.coli JM101 (pCK 9-2) strain was detected as a recombinant plasmid DNA having a smaller distance of movement than the E.coli JM101 (pCK 9). Thus, there was obtained a recombinant plasmid pCK 9-3 DNA having three CK genes arranged in series under control of the lactose promoter inside the sectioned part of KpnI.

(5) Isolation of recombinant plasmid pCK 9-3 DNA

From E.coli JM101 (pCK 9-3), 0.8 mg of the recombinant plasmid pCK 9-3 DNA was obtained by the method described in (1) of Item [1] .

[10] Production of recombinant bacteriophage λEN501S-CK(lacP)3 having CK gene segment inserted under control of DNA arrangement necessary for translation at sectioned part of endonucleotide present in the part of genetic information for production of coating protein of bacteriophage EN501S-Tc

A connecting reaction was effected between 10 µg of the bacteriophage EN501S-Tc DNA obtained in (3) of Item [2] and 30 µg of the recombinant plasmid pCK 9-3 DNA obtained in (5) of Item [9] by mixing the two DNA's mentioned above, sectioning the resultant mixture with KpnI (produced by Boehringer Mannheim-Yamanouchi K.K.) in the same manner as in Item [3], subjecting the sectioned mixture to the treatment with phenol and the treatment for ethanol precipitation as generally practised, and then allowing 2 units of the T4 DNA ligase (produced by Boehringer Mannheim-Yamanouchi K.K.) to react on the treated

mixture at 4°C for 18 hours.

A lysogen was obtained by enclosing 10 µg of the consequently obtained DNA with a coating protein of λ bacteriophage by the in vitro packaging method similarly to Item [3] thereby preparing bacteriophage particles, allowing 50 µl of a solution of the phage particles and E.coli 1100 ($10^9$/ml, 0.5 µl) added thereto to incubate at 30°C for 6 hours, sprinkling the incubated microorganism on an agar culture medium containing tetracycline (Tc) in a concentration of 0.15 µg/ml, culturing the microorganism at 32°C for 48 hours, and selecting a Tc-tolerant strain.

This lysogen was shaken cultured in a T-Y culture medium at 32°C for 16 hours. 0.5-ml of the resultant culture broth was inoculated to 10 ml of a T-Y culture medium in a conical flask having an inner volume of 150 ml, cultured until the number of Klett units reached about 100, heated to 43°C and shaken at this temperature for 25 minutes, cooled to 37°C and shaken at this temperature for about 3 hours. 1-ml of the culture broth thus obtained was clushed with ultrasonic wave to obtain a cell extract. This cell extract was tested for enzyme activity of CK in accordance with the method of Marvin L. Tanzer et al, "Journal of Biological Chemistry," vol. 234, pages 3201 to 3204 (1959). A strain of high activity was selected.

A recombinant bacteriophage DNA was obtained by culturing the lysogen of high activity obtained as described above and treating the cultured lysogen in the manner described in Item [3]. This DNA was dissolved in 20 µl of a solution of the composition of 10 mM Tris-HCl (pH 7.5)/1 mM EDTA. In 30 µl of a solution of the composition of 10 mM Tris-HCl (pH 7.5)/10 mM $MgCl_2$/1 mM dithioerythritol, 4 µl of the resultant solution and 10 units of KpnI (produced by Boehringer Mannheim-Yamanouchi K.K.) added thereto were left standing at 37°C for 2 hours for the purpose of digestion and the digested microorganism was subjected to agarose electrophoresis to determine size of DNA segment.

As the result, the lysogen of high CK activity was found to retain about 4.5 Kb of DNA segment originating in pCK 9-3.

The recombinant bacteriophage EN501S-CK (lacP)3 was produced by separating the E.coli 1100 (EN501S-CK(lacP)3) as described above.

[11] Prepration of recombinant plasmid pAK122E DNA

By isolating a recombinant plasmid pAK122 DNA by the method described in (1) of Item [1] from the E.coli JM101 (pAK122) (FERM BP-1534) retaining the recombinant plasmid pAK122 DNA, there was obtained 1 mg of the DNA in purified form.

A plasmid pAK123E DNA possessing two sectioned parts of EcoRI on the plasmid DNA was obtained by causing reaction of 5 µg of the recombinant plasmid pAK122 DNA with 5 units of PstI (produced by Boehringer Mannheim-Yamanouchi K.K.) in 20 µl of a solution containing 50 mM Tris-HCl (pH 7.5)/10 mM $MgCl_2$/1 mM dithioerythritol/100 mM NaCl at 37°C for 1 hour, subjecting the resultant reaction solution to a treatment for precipitation as generally practised, dissolving the precipitate in 17 µl of water, combining the resultant aqueous solution with 0.3 µg of PstI linker, and allowing 1 unit of the T4 DNA ligase to react upon the mixture at 20°C for 16 hours.

A transformed strain, i.e. E.coli 1100 (pAK123E), attaining growth on a T-Y culture medium containing Ap in a concentration of 20 µg/ml was obtained by causing the aforementioned E.coli treated in advance with calcium chloride to be transformed by the method described in (1) of Item [1] using the recombinant plasmid pAK123E DNA mentioned above.

[12] Preparation of recombinant bacteriophage EN1121SΔRzKm-ACK having ACK gene segment inserted under control of DNA arrangement necessary for translation at sectioned part of endonuclease present in DNA part ranging in downstream area to cohesive end due to anaphasic promoter of bacteriophage $\lambda cI_{857}$1121SΔRzKm

A connecting reaction was effected by extracting $\lambda cI_{857}$1121SΔRzKm DNA by the conventional method from the bacteriophage obtained in (7) of Item [5], mixing 10 µg of this DNA with 3 µg of the recombinant plasmid pAK123E DNA obtained in [3], placing the resultant mixture in 50 µl of a solution of the composition of 50 mM Tris-HCl (pH 7.4)/100 mM NaCl/10 mM $MgSO_4$, causing 50 units of EcoRI to react on the resultant mixture at 37°C for 2 hours, subjecting the resultant reaction solution to the conventional treatments of phenol extaction and ethanol precipitation thereby giving rise to a precipitate, and allowing the precipitate to react on 8 µl of a solution of the composition of 50 mM Tris-HCl (pH 7.4)/10 mM dithiothreitol/10 mM $NaCl_2$/0.1 mM ATP at 4°C for 18 hours.

Bacteriophage particles were prepared by enclosing 10 µg of the consequently obtained DNA by the in vitro packaging method. A 50-µl solution of the phage particles and E.coli 1100 ($10^9$/ml, 0.5µl) added

thereto were left standing at 30°C for 2 hours for the purpose of incubation. The incubated mixture was sprinkled on a T-Y agar culture medium containing Kanamycin (Km) in a concentration of 10 μg/ml and cultured at 32°C for 24 hours.

From the strains cultured and grown as described above, the lysogen E.coli 1100 (EN1121SORzKm-ACK1) due to the recombinant bacteriophage DNA retaining ACK on the bacteriophage $\lambda cI_{857}$1121SΔRzKm DNA was selected in accordance with the method described in Item [6].

[13] Production of bacteriophage 501S-CK(lacP)3ACK retaining CK and ACK genes on one and same bacteriophage DNA

(1) preparation of E.coli 1100 (501CK(lacP)3 ACK

From the E.coli 1100 (EN501S-CK[lacP)3) obtained as described in Item [10], a bacteriophage EN501S-CK(lacP)3 DNA was extracted by the method described in (3) of Item [2]. Similarly, a bacteriophage DNA was extracted by the method described in (3) of Item [2] from the E.coli 1100 obtained as described in Item [12].

Then, a connecting reaction was effected by mixing 10 μg of the bacteriophage EN501CK(lacP)3 DNA with 10 μg of the bacteriophage EN1121SΔRzKm-ACK1 DNA, placing the resultant mixture in 50 μl of a solution of the composition of 10 mM Tris-HCl (pH 7.5)/10 mM $MgCl_2$/1 mM dithioerythritol/50 mM NaCl, allowing 50 units of NheI (produced by Boehringer Mannheim-Yamanouchi K.K.) to react upon the resultant solution at 37°C for 2 hours, then subjecting the resultant reaction solution to the conventional treatments of phenol extraction and ethanol precipitation thereby giving rise to a precipitate, placing the precipitate in 8 μl of a solution of the composition of 50 mM Tris-HCl (pH 7.4)/10 mM $MgCl_2$/10 mM dithiothreitol/0.1 mM ATP, and allowing 2 units of the T4 DNA ligase to react upon the resultant solution at 4°C for 18 hours.

A lysogen was obtained by enclosing 10 μg of the consequently obtained DNA with the coating protein of λ bacteriophage by the in vitro packaging method thereby preparing bacteriophage particles, allowing 50 μl of a solution of the phage particles and E.coli 1100 ($10^9$/ml, 0.5 μl) added thereto to stand at 30°C for 2 hours for the purpose of incubation, sprinkling the incubated mixture on a T-Y culture medium containing Km in a concentration of 10 μg/ml, and culturing at 32°C for 24 hours.

The lysogen containing the recombinant DNA was selected as a strain attaining growth in a culture medium containing Km in a concentration of 10 μg/ml, attaining no growth in a culture medium containing Tc in a concentration of 1.5 μg/ml, and lacking an ability to form a plaque. The lysogen thus selected was cultured as described in Item [6]. The strains in the resultant culture broth were tested for enzyme activity of CK. A strain of high activity was selected.

A recombinant bacteriophage DNA was obtained by culturing the lysogen of high activity obtained as described above and treating the cultured lysogen as described in Item [3].

The E.coli 1100 (501CK(lacP)3ACK) was separated as described above. The transformed strain has been deposited at Fermentation Research Institute, Agency of Industrial Science and Technology as FERM BP-2734.

(2) CK and ACK activities and creatine phosphoric acid forming ability of cell bodies obtained by culturing lysogen due to recombinant bacteriophage λEN501S-CK(lacP)3 DNA and bacteriophage 501CK(lacP)3 ACK DNA.

The CK and ACK activities possessed by the lysogens, i.e. E.coli 1100 (EN501S-CK(lacP)3) and E.coli 1100 (EN501S-CK(lacP)3ACK), obtained as described above are shown in Table 4.

The enzyme activity was determined of a cell extract prepared from cell bodies cultured in a T-Y culture medium in accordance with the method described in Item [6].

The enzyme activity was tested by the method described in "Journal of Biological Chemistry," vol. 234, pages 3201 to 3204 (1959) and "Journal of Bacteriology," vol. 144, pages 672 to 682 (1980).

Table 4

| Kind of strain | Enzyme activity | |
|---|---|---|
| | μ•mol/min./mg of protein | |
| | CK | ACK |
| E.coli 1100 (EN501S-CK(lacP)3) (Control) | 0.52 | 1.65 |
| E.coli 1100 (EN501S-CK(lacP)3ACK1) (This invention) | 0.59 | 83.10 |

The E.coli 1100 (EN501S-CK(lacP)3) and the E.coli 1100 (EN501S-CK(lacP)3ACK1) were each cultured in a T-Y culture medium in accordance with the method described in Item [6]. The cell bodies consequently obtained were frozen, melted, and treated with ultrasonic wave for the purpose of bacteriolysis. The product was used as a crude enzyme solution.

The crude enzyme solution and 20 mM creatine, 20 mM magnesium chloride, 20 or 0.2 mM ATP, 20 or 39.8 mM acetyl phosphoric acid, and 100 mM glycine-NaOH (pH 9.0) buffer solution added thereto were left reacting at 30°C for 1 hour. The amount of creatine phosphoric acid consequently formed in the reaction solution is shown in conversion (%) from creatine in Table 5.

Table 5

| Kind of strain | 20 mM ATP 20 mM acetyl phosphoric acid | 0.2 mM ATP 39.8 mM acetyl phosphoric acid |
|---|---|---|
| E.coli 1100 (EN501S-CK(lacP)3) (Control) | 6.9 | 5.4 |
| E.coli 1100 (EN501S-CK(lacP)3ACK1) (This invention) | 16.9 | 17.2 |

## Claims

1. A recombinant DNA, characterized by having inserted in a vector DNA a DNA containing a gene coding for an enzyme capable of synthesizing a physiologically active substance by conversion of ATP to ADP and a DNA containing a gene coding for acetate kinase.

2. A recombinant DNA according to claim 1, wherein said enzyme capable of synthesizing a physiologically active substance by conversion of ATP to ADP comprises a γ-glutamyl-L-cysteine synthetase and glutathione synthetase.

3. A recombinant DNA according to claim 1, wherein said enzyme capable of synthesizing a physiologically active substance by conversion of ATP to ADP comprises creatine kinase.

4. A method for the production of a physiologically active substance, characterized by culturing in a culture medium a physiologically active substance-producing microorganism of genus Escherichia containing a recombinant DNA as claimed in claim 1, 2 or 3 and collecting said physiologically active substance from the resultant culture broth.

5. A method according to claim 4, wherein said enzyme comprises a γ-glutamyl-L-cysteine synthetase and glutathione synthetase and the physiologically active substance is glutathione.

6. A method according to claim 5 wherein a contact catalysis is induced between the cultured cell bodies of said microorganism and/or processed bodies of said microorganism on the one hand and glutamic acid, cysteine, glycine, adenosine-5'-triphosphoric acid, acetylphosphoric acid, and magnesium ion on the other hand.

7. A method according to claim 4 wherein said enzyme comprises creatine kinase and the physiologically active substance is creatine phosphoric acid.

**Patentansprüche**

1. Rekombinante DNA, gekennzeichnet durch ein DNA Insert in einer Vektor DNA, das ein Gen enthält, welches für ein Enzym kodiert, das zur Synthese einer physiologisch aktiven Substanz unter Umwandlung von ATP in ADP fähig ist, und durch eine DNA, die ein für Acetatkinase kodierendes Gen enthält.

2. Rekombinante DNA nach Anspruch 1, worin dieses zur Synthese einer physiologisch aktiven Substanz unter Umwandlung von ATP in ADP fähige Enzym γ-Glutamyl-L-cysteinsynthetase und Glutathionsynthetase umfaßt.

3. Rekombinante DNA nach Anspruch 1, worin dieses zur Synthese einer physiologisch aktiven Substanz unter Umwandlung von ATP zu ADP fähige Enzym Kreatinkinase umfaßt.

4. Verfahren zur Herstellung einer physiologisch aktiven Substanz, gekennzeichnet durch Kultivierung eines Mikroorganismus der Gattung Escherichia, der eine physiologisch aktive Substanz bildet und eine rekombinante DNA nach Anspruch 1, 2 oder 3 enthält, in einem Kulturmedium, und Gewinnung dieser physiologisch aktiven Substanz aus der entstehenden Kulturbrühe.

5. Verfahren nach Anspruch 4, worin dieses Enzym γ-Glutamyl-L-cysteinsynthetase und Glutathionsynthetase umfaßt und die physiologisch aktive Substanz Glutathion ist.

6. Verfahren nach Anspruch 5, worin eine Kontaktkatalyse zwischen den kultivierten Zellkörpern dieses Mikroorganismus und/oder verarbeiteten Zellkörpern dieses Mikroorganismus auf der einerseits einen Seite und Glutaminsäure, Cystein, Glycin, Adenosin-5'-triphosphorsäure, Acetylphosphorsäure und Magnesiumionen auf der andererseits anderen Seite ausgelöst wird.

7. Verfahren nach Anspruch 4, worin dieses Enzym Kreatinkinase umfaßt und die physiologisch aktive Substanz Kreatinphosphorsäure ist.

**Revendications**

1. ADN recombinant, caractérisé en ce qu'il a, inséré dans un ADN vectoriel, un ADN contenant un gène codant pour une enzyme, capable de synthétiser une substance physiologiquement active par conversion de ATP en ADP et un ADN contenant un gène codant pour l'acétate kinase.

2. ADN recombinant selon la revendication 1, où ladite enzyme capable de synthétiser une substance physiologiquement active par conversion de ATP en ADP comprend une γ-glutamyl-L-cystéine synthétase et de la glutathione synthétase.

3. ADN recombinant selon la revendication 1, où ladite enzyme capable de synthétiser une substance physiologiquement active par conversion de ATP en ADP comprend la créatine kinase.

4. Méthode pour la production d'une substance physiologiquement active, caractérisée par la mise en culture, dans un milieu de culture, d'un microorganisme produisant une substance physiologiquement active du genre Escherichia contenant un ADN recombinant selon l'une quelconque des revendications 1, 2 ou 3 et la récupération de ladite substance physiologiquement active du bouillon de culture résultant.

5. Méthode selon la revendication 4, où ladite enzyme comprend une $\gamma$-glutamyl-L-cystéine synthétase et une glutathione synthétase et la substance physiologiquement active est la glutatione.

6. Méthode selon la revendication 5, où une catalyse par contact est induite entre les corps des cellules en culture dudit microorganisme et/ou les corps traités dudit microorganisme d'une part et l'acide glutamique, la cystéine, la glycine, l'acide adénosine-5'-triphosphorique, l'acide acétylphosphorique et l'ion magnésium d'autre part.

7. Méthode selon la revendication 4, où ladite enzyme comprend la créatine kinase et la substance physiolologiquement active est l'acide créatine phosphorique.

FIG. 1

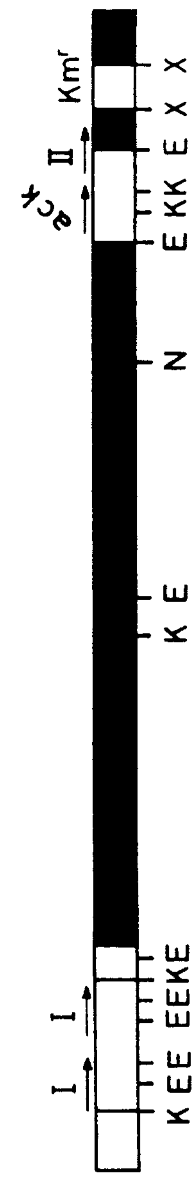


501GI(lacP)2ACK1GII(lacp)1